(19) Europäisches Patentamt
European Patent Office
Office européen des brevets

(11) Veröffentlichungsnummer: **0 001 585**
**B1**

(12) # EUROPÄISCHE PATENTSCHRIFT

(45) Veröffentlichungstag der Patentschrift: **22.07.81**

(51) Int. Cl.³: **C 07 D 487/14,**
**A 61 K 31/55**

(21) Anmeldenummer: **78101052.5**

(22) Anmeldetag: **02.10.78**

(54) **Piperazino-pyrrolobenzodiazepine, Verfahren zu ihrer Herstellung sowie pharmazeutische Präparate enthaltend diese Verbindungen.**

(30) Priorität: **05.10.77 US 839696**

(43) Veröffentlichungstag der Anmeldung:
**02.05.79 Patentblatt 79/9**

(45) Bekanntmachung des Hinweises auf die Patenterteilung:
**22.07.81 Patentblatt 81/29**

(84) Benannte Vertragsstaaten:
**BE CH DE FR GB LU NL SE**

(56) Entgegenhaltungen:
**DE - A - 2 712 309**
**US - A - 3 763 183**
**US - A - 3 984 562**
**US - A - 3 985 732**
**US - A - 4 073 784**

(73) Patentinhaber: **CIBA-GEIGY AG**
**Patentabteilung Postfach**
**CH-4002 Basel (CH)**

(72) Erfinder: **Wasley, Jan W.F.**
**55 Floral Street**
**Chatham New Jersey 07928 (US)**

(74) Vertreter: **Zumstein sen., Fritz, Dr. et al,**
**Bräuhausstrasse 4**
**D-8000 München 2 (DE)**

**EP 0 001 585 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Piperazino-pyrrolobenzodiazepine, Verfahren zu ihrer Herstellung, sowie pharmazeutische Präparate enthaltend diese Verbindungen

Die Erfindung betrifft neue 1,3,4,14b-Tetrahydro-2H,10H-pyrazino[1,2-a]pyrrolo[2,1-c]-[1,4]benzodiazepine der allgemeinen Formel I

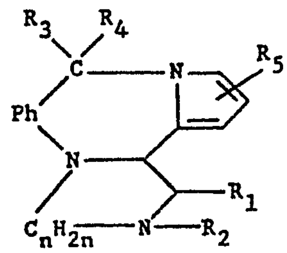

(I)

worin jedes der Symbole $R_1$, $R_3$ und $R_4$ Wasserstoff oder Niederalkyl bedeutet, $R_2$ für Wasserstoff, Niederalkyl, Höheralkyl, Niederalkenyl, Niederalkynyl, Cycloalkyl oder Cycloalkenyl mit jeweils 3 bis 7 Ringgliedern oder (Cycloalkyl, Hydroxy, Amino, Mono- oder Di-niederalkylamino, Carboxy, niederes Carbalkoxy, Carbamoyl, Mono- oder Di-niederalkyl-carbamoyl, HPh, Niederalkanoyl oder HPhCO)-niederalkyl steht, Ph 1,2-Phenylen bedeutet, welches durch höchstens 2 Substituenten der Gruppe bestehend aus Niederalkyl, Niederalkoxy, Niederalkylthio, Halogen und Trifluormethyl substituiert sein kann, $C_nH_{2n}$ für Niederalkylen steht, welches die zwei Stickstoffatome durch 2 oder 3 Kohlenstoffatome trennt, und $R_5$ Wasserstoff, Niederalkyl, Carboxy, niederes Carbalkoxy oder (Hydroxy, Amino, Mono- oder Di-niederalkylamino)-niederalkyl bedeutet; die Niederalkoxycarbonyl-, Niederalkanoyl-, Höheralkanoyl-, Adamantoyl-, Carbamoyl-, Mono- oder Di-niederalkylcarbamoyl Cycloalkyl-carbonyl-, mit 3 bis 7 Ringgliedern, oder HPhCO-Derivate, die 2-N-Oxide, die 2-Niederalkyl- oder 2-HPh-Niederalkyl-quaternären Ammoniumderivate und ihre Salze, insbesondere therapeutisch verwendbaren Salze mit Säuren oder Basen.

Eine Niederalkylgruppe $R_1$, $R_2$, $R_3$, $R_4$, $R_5$ und/oder eine solche in einem substituierten 1,2-Phenylen Ph oder Phenyl HPh-Rest vorhandene. Gruppe ist vor allem Methyl, aber auch Aethyl, n- oder iso-(Propyl, Butyl, Pentyl, Hexyl oder Heptyl), z.B. 2-Methylpropyl oder 3-Methylbutyl. Eine höhere Alkylgruppe $R_2$ ist z.B. n-(Octyl, Decyl, Dodecyl, Hexadecyl oder Octadecyl).

Eine Niederalkenyl- oder Niederalkynylgruppe $R_2$ ist vorzugsweise eine solche, in welcher die mehrfache Bindung vom Stickstoffatom durch mindestens zwei Kohlenstoffatome getrennt ist, z.B. Allyl, 2- oder 3-Butenyl oder 3-Methyl-2-butenyl; Propargyl, 2- oder 3-Butynyl.

Eine 3 bis 7 Ringglieder enthaltende Cyclalkyl-, Cycloalkenyl- oder Cycloalkyl-niederalkylgruppe $R_2$ ist vorzugsweise Cyclopropyl, Cyclopentyl, Cyclohexyl oder Cycloheptyl; 2- oder 3-Cyclopentenyl- oder 2- oder 3-Cyclohexenyl; (Cyclopropyl, Cyclobutyl oder Cyclopentyl)-methyl oder -äthyl.

Eine (Hydroxy, Amino, Mono- oder Di-niederalkylamino, Carboxy, Carbalkoxy, Carbamoyl, Mono- oder Di-niederalkyl-carbamoyl oder Niederalkanoyl)-niederalkylgruppe $R_2$ ist vorzugsweise eine solche, worin die Heteroatome (O oder N) vom Ringstickstoffatom durch mindestens 2 Kohlenstoffatome getrennt sind, z.B. 2-(Hydroxy, Amino, Methylamino, Aethylamino, Dimethylamino, Diäthylamino, Carboxy, Carbomethoxy, Carbäthoxy, Carbamoyl, Mono- oder Dimethylcarbamoyl, Acetyl oder Propionyl)-äthyl oder -propyl, 3-Hydroxy-propyl oder (Carboxy, Carbomethoxy, Carbäthoxy, Carbamoyl, Mono- oder Dimethylcarbamoyl oder Acetyl)-methyl.

Ein 1,2-Phenylenrest Ph oder die (Phenyl oder Benzoyl, d.h. HPh oder HPhCO)-niederalkylgruppe $R_2$ ist im benzolring vorzugsweise unsubstituiert oder monosubstituiert durch eine der genannten Substituenten, z.B. Methyl oder Aethyl; Methoxy, Aethoxy oder iso-Propoxy; Methylthio oder Aethylthio; Fluor, Chlor, Brom oder Trifluormethyl.

Eine Niederalkylengruppe $C_nH_{2n}$ ist insbesondere Aethylen, aber auch 1,2- oder 1,3-Propylen oder 1,2-, 1,3- oder 2,3-Butylen.

Eine Carboniederalkoxygruppe oder eine (Hydroxy, Amino, Mono- oder Di-niederalkylamino)-niederalkylgruppe $R_5$ ist vorzugsweise Carbomethoxy oder Carbäthoxy oder (Hydroxy, Amino, Mono- oder Dimethylamino, Mono- oder Diäthylamino)-methylgruppe.

Die genannten Acylderivate von Verbindungen der Formel I sind entweder von solchen, in denen $R_2$ Wasserstoff, aber auch von solchen, in denen $R_2$ und/oder $R_5$ (Hydroxy, Amino oder Mono-niederalkylamino)-niederalkyl bedeutet, abgeleitet. Diese Derivate sind somit Amide oder Ester, Welche von niederen oder höheren aliphatischen oder cycloaliphatischen Säuren, z.B. Essig-, Propion-, Butter-, Pivalin-, Dekan-, Palmitin, Hexahydrobenzoesäure oder Adamantylcarbonsäure, Carbaminsäure, Mono- oder Dimethyl-, Mono- oder Diäthylcarbaminsäure oder Kohlensäure; Benzoe-, Toluol, Amissäure oder Halogenbenzoesäuren abgeleitet sind.

Die N-Oxide, Niederalkyl- oder Phenyl-niederalkyl quaternären Derivate sind vorzugsweise von Verbindungen der Formel I abgeleitet, in welchen $R_2$ nicht Wasserstoff ist und in welchen lediglich das Stickstoffatom in 2-Stellung funktionalisiert ist. Die Anionen oder quaternären Verbindungen und der Säureadditionssalze sind vorzugsweise solche von therapeutisch verwendbaren Säuren, z.B. der weiter unten genannten Säuren. Verbindungen der Formel I, welche eine Carboxygruppe im Rest $R_2$ und/oder $R_5$ aufweisen, bilden auch Salze mit solchen Basen wie z.B. Ammoniak, Mono-, Di- oder Triniederalkylamine, niedere Alkylenamine, Morpholin, Piperazin, Pyridin oder Niederalkylderivaten der genannten cyclischen Basen; Alkalimetall- oder Erdalkalimetallhydroxyden.

Der Ausdruck "nieder" definiert in den oben oder nachfolgend genannten organischen Resten oder Verbindungen solche mit höchstens 7, vorzugsweise bis 4, in erster Linie mit einem oder zwei Kohlenstoffatomen. Der Ausdruck "höher" definiert Reste oder Verbindungen mit 8 bis 20, vorzugsweise 8 bis 16 Kohlenstoffatomen.

Aus der US—PS 3 984 562 waren tricyclische Benzodiazepinderivate bekannt, die einen Piperazino-niederalkanoyl-Rest sowie einen gesättigten Pyrrolidinring aufweisen und die eine beruhigende Wirkung besitzen. Demgegenüber unterscheiden sich die erfindungsgemäßen Verbindungen ganz wesentlich in ihrer Struktur (tetracyclisch, kein Piperazino-niederalkanoylrest, ungesättigter Pyrrolring), aber auch hinsichtlich ihrer Wirkung.

Die Verbindungen der Erfindung zeigen wertvolle pharmakologische Eigenschaften, in erster Linie antidepressive, aber auch analgetische, antihistaminische und antiserotonergische Wirkungen. Diese können in Tierversuchen, vorzugsweise an Säugetieren, wie Mäusen, Ratten, Meerschweinchen und Affen, als Testobjekte nachgewiesen werden. Die neuen Verbindungen können ihnen enteral oder parenteral, vorzugsweise oral, subkutan, intravenös oder intraperitoneal, z.B. durch Gelatine-Kapseln oder in Form von Stärke enthaltenden Suspensionen bzw. wässerigen Lösungen, verabreicht werden. Die verwendete Dosis kann in einem Bereich von ungefähr zwischen 0,01 und 100 mg/kg/Tag, vorzugsweise ungefähr 0,05 und 5e mg/kg/Tag, insbesondere ungefähr 0,1 und 0,5 mg/kg/Tag liegen. Die antidepressiven Eigenschaften können an der Maus durch das Aufheben der Clonidin-Analgesie, nachgewiesen werden. In diesem Testsystem werden die Verbindungen der Erfindung als wässerige Lösungen oral oder intraperitoneal an Gruppen von mindestens 10 männlichen Mäusen verabreicht und 30 Minuten später wird ihnen 0,1 mg/kg Clonidin intubiert. Nach 20 Minuten injiziert man den Tieren 3,75 mg/kg Phenyl-p-benzochinon intraperitoneal, und 5—15 Minuten nach der Injektion stellt man die Anzahl der sich windenden Tiere fest. Jedes sich nicht krümmende Tier wird als positiv bezeichnet und der $ED_{50}$-Wert für die Kombination des geprüften Wirkstoffes und des Clonidins durch die Berkson Logit-Methode aus der Anzahl der positiv reagierenden Tiere bestimmt.

Die genannten analgetischen Effekte können ähnlich durch den Phenyl-p-benzochinon-Krümmungstest nachgewiesen werden. Gemäss diesem Test erhalten Gruppen von mindestens 10 männlichen Mäusen oral oder intraperitoneal die Verbindungen der Erfindung und nach 50 Minuten 3,75 mg/kg Phenyl-p-benzochinon intraperitoneal. Nach 5—15 Minuten wird wieder die Anzahl der sich krümmenden Tiere bestimmt und der $ED_{50}$-Wert für die sich nicht krümmenden Tiere durch die Berkson Logit-Methode bestimmt.

Schliesslich können die antihistaminischen Eigenschaften in vitro gemäss Chasin et al., J. Neurochem. *22,* 1031 (1974) nachgewiesen werden. Homogenate eines zellfreien Präparats werden vorher mit $^3$H-Adenin inkubiert, wobei endogenes $^3$-H-Adenosin-triphosphat gebildet wird. Die Homogenate werden dann mit 50 mikromolarem Histamin inkubiert, um die synthese von $^3$H-cyclischem Adenosinmonophosphat zu aktivieren. Dieses Vorgehen wird in Gegenwart oder Abwesenheit der Testverbindung unternommen, wobei man Konzentrationen zwischen 0,01 und 100 Mikromolen einsetzt. ist die geprüfte Substanz aktiv, so wird die Histamin-Aktivierung der Adenylat-Cyclase gehemmt.

Die Verbindungen der Erfindung können dementsprechend als Antidepressiva, Analgetika und Antihistaminpräparate, z.B. in der Behandlung oder Handhabung von mentalen Depressionen, Migräne und/oder von allergischen Zuständen verwendet werden. Sie können auch also Zwischenprodukte zur Herstellung von anderen wertvollen Produkten, insbesondere von pharmakologisch wirksamen Präparaten eingesetzt werden.

Bevorzugte Verbindungen sind diejenigen der Formel I, worin jedes der Symbole $R_1$, $R_3$ und $R_4$ Wasserstoff bedeutet, $R_2$ für Wasserstoff, Niederalkyl, Niederalkenyl, Niederalkynyl, Hydroxy-niederalkyl, Cycloalkyl oder Cycloalkyl-niederalkyl mit jeweils 3 bis 7 Ringkohlenstoffatomen steht, Ph 1,2-Phenylen, (Niederalkyl)-1,2-phenylen, (Niederalkoxy)-1,2-phenylen, (Niederalkylthio)-1,2-phenylen, (Halogen)-1,2-phenylen oder (Trifluoromethyl)-1,2-phenylen bedeutet, n eine ganze Zahl von 2 oder 3 ist, und $R_5$ Wasserstoff, Niederalkyl oder Hydroxy-niederalkyl bedeutet, ihre Niederalkanoyl-, Adamantoyl-, Carbamoyl-, Mono- oder Di-niederalkylcarbamoyl oder HPhCO-Derivate, 2-N-Oxide, 2-Niederalkyl- oder 2-HPh-Niederalkyl quaternären Derivate und ihre therapeutisch verwendbaren Säureadditionssalze.

Besonders hervorzuheben sind die Verbindungen der allgemeinen Formel II

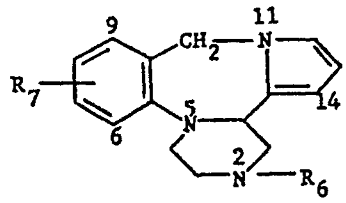

(II)

worin $R_6$ Wasserstoff, Niederalkyl, Niederalkenyl, Niederalkynyl, Hydroxy-niederalkyl oder Cycloalkylniederalkyl mit 3 bis 7 Ringgliedern bedeutet, und $R_7$ für Wasserstoff, Halogen oder Trifluormethyl steht, und ihre therapeutisch verwendbaren Säureadditionssalze.

Besonders bevorzugt sind die Verbindungen der allgemeinen Formel II, worin $R_6$ Alkyl, Alkenyl, Alkynyl oder Hydroxyalkyl mit jeweils bis 4 Kohlenstoffatomen oder Cyclopropylmethyl bedeutet, und $R_7$ für Fluor oder Chlor, vorzugsweise in 7-Stellung steht, und ihre therapeutisch verwendbaren Säureadditionssalze.

Die Verbindungen der Erfindung können nach an sich bekannten Methoden, vorzugsweise dadurch hergestellt werden, dass man eine Verbindungen der allgemeinen Formel III

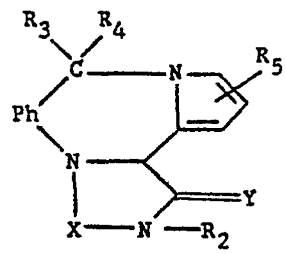

(III),

worin X Niederalkylen, Mono- oder Dioxo-niederalkylen bedeutet, welches die zwei Stickstoffatome durch 2 oder 3 Kohlenstoffatome trennt und worin Oxo zu dem mit dem Stickstoffatom benachbarten Kohlenstoffatom gebunden ist, und Y für Oxo, zwei Wasserstoffatome oder Wasserstoff und Niederalkyl steht, mit der Massgabe, dass mindestens eine Oxogruppe in X oder Y vorhanden ist, reduziert, und, wenn erwünscht, eine erhaltene Verbindung der Formel I in eine andere erfindungsgemässe Verbindung umwandelt.

Die Reduktion der genannten Lactame der Formel III wird vorzugsweise mit einfachen Hydriden oder komplexen Leichtmetallhydriden, wie Boranen oder Alan, in erster Linie mit Alkalimetallaluminiumhydriden oder Alkalimetallniederalkoxyhydriden, z.B. Lithiumaluminiumhydrid, Natrium-tri-t.-butoxy-aluminiumhydrid oder Natrium-bos-(2-methoxy-äthoxy)-aluminiumhydrid, durchgeführt.

Die Ausgangsstoffe können gemäss den für ihre bekannten Analoga beschriebenen Methoden oder wie in den Beispielen illustriert hergestellt werden.

So können die Ausgangsstoffe durch Umsetzung entsprechender Verbindungen der Formel III, in welchen X zwei Wasserstoffatome, bedeutet, von welchen jedes an ein anderes Stickstoffatom gebunden ist, mit reaktionsfähigen Derivaten entsprechender Glykole, Glykolsäuren oder Dicarbonsäuren, wie ihren Niederalkylestern, Halogeniden oder Anhydriden, oder mit reaktionsfähigen Estern der genannten Glykole oder Glykolsäurederivate, z.B. Estern von Halogenwasserstoffsäuren oder aromatischen Sulfonsäuren, wie 1,2-Dibromäthan oder -propan, Bromessigsäure-äthylester oder -propylester, Tosyloxyessigsäure-äthylester, Oxalsäure-diäthylester oder Malonsäure-diäthylester, Oxalsäure-hemi-ethylester-chlorid, Oxalylbischlorid oder Malonsäure-anhydrid hergestellt werden. Die genannten der Formel III entsprechenden Vorstufen der Ausgangsstoffe können analog zu Il Farmaco, Ed. Sc. *24,* Fasc. 3, S. 276 oder wie in den Beispielen illustriert, erhalten werden.

Die erhaltenen Verbindungen der Erfindung können, wenn erwünscht oder notwendig, nach an sich bekannten Methoden ineinander übergeführt werden. So lassen sich z.B. erhaltene Verbindungen der Formel I, in welchen $R_2$ Wasserstoff bedeutet, und/oder $R_5$ für (Amino oder Mononiederalkylamino)-niederalkyl steht, oder ihre Alkalimetall-, z.B. Natriumsalze, durch Umsetzung mit reaktionsfähigen Estern von unsubstituierten oder entsprechend substituierten aliphatischen oder araliphatischen Alkoholen, wie Methanol, Aethanol, Allylalkohol, Propargylalkohol oder Benzylalkohol, je nach der verwendeten Mol-Menge des Alkylierungsmittels, in die entsprechenden N-substituierten Verbindungen bzw. in die quaternären Ammoniumderivate, überführen. Die reaktionsfähigen Ester erhält man durch Veresterung der Alkohole mit starken anorganischen oder organischen Säuren, in erster Linie mit einer Halogenwasserstoffsäure, z.B. Chlorwasserstoff- Bromwasserstoff- oder Jodwasserstoffsäure, Schwefelsäure oder einer aromatischen Sulfonsäure, z.B. p-Toluolsulfonsäure oder m-Brombenzolsulfonsäure.

Umgekehrt können erhaltene N-alkylierte Verbindungen in N-unsubstituierte Verbindungen, z.B. durch katalytische Hydrogenolyse von N-Benzyl-Verbindungen, umgewandelt werden. Setzt man N-Niederalkyl, N-Niederalkenyl- oder N—($CH_2$—HPh)-Derivate mit Halogenameisensäure-niederalkylestern, z.B. Chlorameisensäure-äthylester um, so werden N-Acylderivate erhalten. Die Letztern können in die N-unsubstituierten Verbindungen, vorzugsweise in solche, in denen $R_2$ Wasserstoff ist, z.B. mit wässerigen Basen, z.B. mit Alkalimetallhydroxylösungen, übergeführt werden. Andere Acylderivate, entweder Amide oder Ester, können hergestellt werden, indem man Verbindungen der Formel I, worin $R_2$ Wasserstoff bedeutet oder $R_2$ und/oder $R_5$ für (Hydroxy, amino oder Mono-niederalkylamino)-niederalkyl stehen, mit entsprechenden Säurederivaten, z.B. Halogeniden, einfachen oder aktivierten Estern, wie Alkyl oder Cyan-alkylestern, Anhydriden oder Cyanaten umsetzt. Erhaltene Ester können gleich wie für die N-Acylderivate beschrieben, hydrolysiert werden. Erhaltene ungesättigte Verbindungen, z.B. in welchen $R_2$ Niederalkenyl, Niederalkynyl oder Cycloalkenyl bedeutet, können wie oben beschrieben mit katalytisch aktiviertem Wasserstoff hydriert werden. Erhaltene Ester können auch durch Umsetzung mit Ammoniak oder entsprechenden primären oder sekundären Aminen in Amid-(Carbamoyl)-Verbindungen, oder durch Reduktion mit komplexen Leichtmetallhydriden, z.B. Lithiumaluminiumhydrid in die entsprechenden Alkohole umgewandelt werden.

Erhaltene Verbindungen der Formel I, worin $R_3$ und/oder $R_4$ Wasserstoff bedeutet, können in die entsprechenden Niederalkylderivate durch Metallisierung mit reaktionsfähigen organischen Metallverbindungen, z.B. n-Butyllithium oder Lithium-diisopropylamid und Umsetzung mit reaktionsfähigen Estern von Niederalkanolen, umgewandelt werden.

Verbindungen der Formel I, worin $R_5$ Wasserstoff bedeutet, können in die entsprechenden 12-Acylderivate, z.B. durch Acylierung mit entsprechenden Derivaten von HO—X—OH, worin X die oben angegebene Bedeutung hat (analog zur Herstellung eines Ausgangsstoffes der Formel III), oder durch Umsetzung, zuerst mit einem Trihalogenacetyl-halogenid und dann mit einem Alkalimetallniederalkoxyd, umgewandelt werden. Erhaltene 12-(Carbalkoxy, Carbalkoxy-carbonyl oder Hydroxymethyl)-Derivate können gemäss den oben angegebenen Methoden hydrolysiert und/oder mit den oben genannten einfachen Hydriden oder komplexen Leichtmetallhydriden zu einer 12-(Methyl, Hydroxymethyl oder 2-Hydroxyäthyl)-Verbindung reduziert werden. Erhaltene tertiäre Stickstoffverbindungen, in welchen $R_2$ nicht Wasserstoff ist, können in ihre N-Oxyde, z.B. mit Wasserstoffsuperoxyd oder mit organischen Persäuren, wie niederen Peralkansäuren oder Perbenzoesäure, z.B. Peressigsäure oder m-Chlor-perbenzoesäure, vorzugsweise bei oder unter Zimmertemperatur mit der letzteren, oder bei Temperaturen bis 100° mit verdünnter Wasserstoffsuperoxydlösung in Gegenwart von Niederalkansäuren, z.B. Essigsäure, übergeführt werden. Man muss dabei vorsichtig vorgehen, insbesondere mit den genannten Persäuren, um Ueberoxydationen bei zu langen Reaktionszeiten zu vermeiden.

Schliesslich können die Verbindungen der Erfindung in Form von freien Basen oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxyd oder Ammoniumhydroxid, basischen Salz oder einem Kationenaustauscher, z.B. mit einem Alkalimetallhydroxyd- oder -carbonat, umgewandelt werden. Säuren, die therapeutisch verwendbare Säureadditionssalze ergeben, sind. z.B. anorganische Säuren, wie Halogenwasserstoffsäure, z.B. Chlorwasserstoff- oder Bromwasserstoffsäure, oder Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol-, Milch-, Aepfel-, Wein-, Zitronen-, Malein-, Fumar-Hydroxymalein-, Brenztrauben-, Phenylessig-, Benzoe-, 4-Aminobenzene-, Anthranil-, 4-Hydroxybenzoe-Salicyl-, 4-Aminosalicyl, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure. Diese oder andere Salze, z.B. die Pikrate, können auch in der Reinigung von freien Basen verwendet werden. Die Basen werden in ihre Salze übergeführt, die Salze abgetrennt und die basen aus den Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Erhaltene isomerengemische von Verbindungen, z.B. solchen der Formeln I bis III, können nach an sich bekannten Methoden, z.B. durch fraktionierte Destillation, Kristallisation und/oder Chromatographie, in die einzelnen Isomeren getrennt werden. Razemische Produkte können in die optische Antipoden, z.B. bei Trennung ihrer diastereoisomeren Salze, z.B. durch fraktionierte Kristallisation der d- oder l-Tartrate getrennt werden.

Die oben genannten Reaktionen werden nach an sich bekannten Methoden, in Gegenwart oder Abwesenheit von Verdünngsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird

und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangstoff in Form eines Salzes oder optisch reinen Antipoden, verwendet wird.

Im Verfahren der vorliegenden Erfindung werden vorteilhfaterweise solche Ausgangsstoffe verwendet, welche zu den im vorstehenden als besonders wertvoll beschriebenen Verbindungen, insbesondere solchen der Formel II, führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur. Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granuliert- oder Dragierverfahren, hergestellt und enthalten von etwa 0,1% bis etwa 75%, insbesondere von etwa 1% bis etwa 50% des Aktivstoffes.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mmHg, durchgeführt.

### Beispiel 1

Eine Suspension von 12,8 g 2-methyl-3,4-dioxo-1,3,4,14b-tetrahydro-2H,10H-pyrazino[1,2-a]pyrrolo[2,1-c] [1,4-benzodiazepin in 460 ml Tetrahydrofuran wird unter Rühren und Kühlen mit Eis mit 200 ml 1-molarem Diboran in Tetrahydrofuran versetzt. Das Gemisch wird eine Stunde unter Rückfluss gekocht, wieder gekühlt und mit 25 ml Essigsäure versetzt. Das Reaktionsgemisch wird eingedampft, der Rückstand in 50 ml einer 30%-igen wässerigen Natriumhydroxydlösung aufgenommen und das Gemisch mit Methylenchlorid extrahiert. Der Extrakt wird getrocknet, eingedampft, der Rückstand in Diäthyläther gelöst, die Lösung filtriert und das Filtrat eingedampft. Man erhält das 2-Methyl-1,3,4,14b-tetrahydro-2H,10H-pyrazino[1,2-a]pyrrolo[2,1-c][1,4]benzodiazepin.

Man löst 9,7 g der letztgenannten Verbindung in einer minimalen Menge Isopropanol und sauert die Lösung mit einer konz. Lösung von 4,45 g Maleinsäure in Isopropanol an. Der erhaltene Niederschlag wird abgetrennt und aus Methanol-Diäthyläther umkristallisiert. Man erhält das entsprechende Mono-maleat, welches bei 176—178° schmilzt.

Man kann 2,35 g des oben genannten Dioxo-Ausgangsstoffes in 50 ml Methylenchlorid auch mit 20 ml 1-molarem Alan in Triäthylamin reduzieren und dann das Gemisch eindampfen. Der Rückstand wird mit Essigsäureäthylester-Diäthyläther trituriert, auf 70 g Silicagel chromatographiert und mit Methanol-Chloroform (1:9) eluiert. Das Eluat wird eingedampft und der Rückstand wie oben beschrieben angesäuert. Man erhält das etwas reinere Maleat, welches bei 180—182° schmilzt.

Der Ausgangsstoff wird wie folgt hergestellt: Ein Gemisch von 54 g Phthalimidkalium, 50 g o-Nitro-benzylchlorid und 120 ml Dimethylformamide wird 3 Stunden unter Rückfluss gekocht und unter Rühren in 900 ml Eiswasser gegossen. Nach 30 Minuten wird es filtriert und der Rückstand mit Wasser gewaschen. Man erhält das N-o-Nitrobenzylphthalimid, welches bei 190—209° schmilzt.

Ein Gemisch von 70 g der letztgenannten Verbindung, 14,6 g Hydrazin-hydrat und 600 ml Aethanol wird 4 Stunden unter Rückfluss gekocht und mit 50 ml konz. Chlorwasserstoffsäure versetzt. Nach 30 Minuten wird das Rekationsgemisch auf Zimmertemperatur gekühlt, filtriert und der Rückstand mit Wasser gewaschen. Das Filtrat wird konzentriert, das wässerige Konzentrat filtriert und das Filtrat mit 3-normaler wässeriger Natriumhydroxydlösung basisch gemacht. Das Gemisch wird mit Diäthyläther extrahiert, der Extrakt getrocknet und eingedampft. Man erhält das o-Nitrobenzylamin.

Eine Lösung von 7,6 g der letztgenannten Verbindung in 25 ml Eisessig wird mit 6,6 g 2,5-Dimethoxy-tetrahydrofuran versetzt und das Gemisch eine Stunde unter Rückfluss gekocht. Das Reaktionsgemisch wird eingedampft, der Rückstand in Eiswasser gegossen und das Gemisch mit Essigsäureäthylester extrahiert. Der Extrakt wird mit gesättigter wässeriger Natriumhydrogencarbonatlösung gewaschen, getrocknet und eingedampft. Der Rückstand wird in Diäthyläther aufgenommen, die Lösung mit Aktivkohle entfärbt, filtriert und eingedampft. Man erhält das 1-(o-Nitrobenzyl)-pyrrol.

Ein Gemisch von 13,42 g der letztgenannten Verbindung, 140 ml Diäthyläther und 6,85 g

Chloracetonitril wird in einem Eis-Salzbad gekühlt und unter Rühren mit Chlorwasserstoffgas gesättigt. Das gesättigte Gemisch wird bei Zimmertemperatur über Nacht gerührt, filtriert und der Rückstand in 100 ml Wasser suspendiert. Die Suspension wird dreimal mit 100 ml Essigsäureäthylester extrahiert, die vereinigten Extrakte werden unter Rühren auf dem Dampfbad zur Auflösung des ausgeschiedenen Materials erwärmt. Die Lösung wird getrocknet und eingedampft. Man erhält das 1-o-Nitrobenzyl-2-chloracetyl-pyrrol.

Eine Lösung von 16,2 g der letztgenannten Verbindung in 450 ml Aethanol wird mit 14,1 g N-Methyl-benzylamin versetzt und das Gemisch 3 Stunden unter Rückfluss gekocht. Das Reaktionsgemisch wird eingedampft, der Rückstand in Methylenchlorid aufgenommen, die Lösung mit gesättigter wässeriger Natriumcarbonatlösung gewaschen, getrocknet, filtriert und eingedampft. Der Rückstand wird mit Diäthyläther trituriert. Man erhält das 1-(o-Nitrobenzyl)-2-(N-methyl-N-benzylaminoacetyl)-pyrrol.

Eine Lösung von 3 g der letztgenannten Verbindung in 30 ml Essigsäure wird bis zur Aufnahme der theoretischen Menge Wasserstoff über 100 mg Platinoxyd bei 2,7 Atmosphären und Zimmertemperatur hydriert. Das Gemisch wird filtriert, das Filtrat eingedampft, der Rückstand in Methylenchlorid-Diäthyläther aufgenommen und die Lösung mit gesättigter wässeriger Natriumhydrogencarbonatlösung gewaschen. Die Lösung wird getrocknet, eingedampft und der Rückstand auf 30 g Silicagel chromatographiert und mit Methanol-Chloroform (1:9) eluiert. Man erhält das 11-(N-Methyl-N-benzylamino-methyl)-10,11-dihydro-5H-pyrrolo[2,1-c][1,4]benzodiazepin, welches bei 147—149° schmilzt.

Eine Lösung von 500 mg der letztgenannten Verbindung in 35 ml Aethanol und 5 ml Eisessig wird über 250 mg eines 5%-igen Palladium-auf-Kohle-Katalysators 7 Stunden bei 40° und 2,7 Atmosphären hydriert. Das Gemisch wird filtriert, das Filtrat eingedampft und der Rückstand in Methylenchlorid aufgenommen. Die Lösung wird mit gesättigter wässeriger Natriumcarbonatlösung gewaschen, die wässerige Phase mit Methylenchlorid extrahiert und die vereinigten organischen Lösungen werden getrocknet und eingedampft. Man erhält das 11-(N-Methylamino-methyl)-10,11-dihydro-5H-pyrrolo[2,1-c][1,4]benzodiazepin.

Ein Gemisch von 300 mg der letztgenannten Verbindung und 232 mg Oxalsäure-diäthylester wird langsam, innerhalb von 45 Minuten auf 140° und innerhalb von 15 Minuten auf 180° erhitzt. Das Gemisch wird 30 Minuten bei der letztgenannten Temperatur gehalten, dann gekühlt, mit Benzol verdünnt, auf Silicagel chromatographiert und mit Methanol-Chloroform (1:9) eluiert. Man erhält das 2-Methyl - 3,4 - dioxo - 1,3,4,14b - tetrahydro - 2H,10H - pyrazino[1,2-a]pyrrolo[2,1-c][1,4]benzodiazepin, welches bei 178—179° schmilzt.

Beispiel 2

Eine Suspension von 315 mg 7-Chlor-2-methy-3,4-dioxo-1,3,4,14b-tetrahydro-2H,10H-pyrazino[1,2-a]pyrrolo[2,1-c][1,4]benzodiazepin in 20 ml Tetrahydrofuran wird mit 6 ml 1-molarem Diboran in Tetrahydrofuran unter Rühren und Eiskühlung versetzt. Das Gemisch wird 2 Stunden unter Rückfluss gekocht, wieder gekühlt und mit 1 ml 6-normaler Chlorwasserstoffsäure versetzt. Das Reaktionsgemisch wird eingedampft, der Rückstand in 5 ml einer 30%-igen wässerigen Natriumhydroxydlösung aufgenommen und das Gemisch mit Methylenchlorid extrahiert. Der Extrakt wird getrocknet, eingedampft, der Rückstand in Diäthyläther gelöst und die Lösung mit 97 mg Maleinsäure in einer minimalen Menge Aceton versetzt. Der erhaltene Niederschlag wird abgetrennt. Man erhält das 7 - Chlor - 2 - methyl - 1,3,4,14b - tetrahydro - 2H,10H - pyrazino[1,2-a]pyrrolo[2,1-c][1,4]benzodiazepin-mono-maleat, welches bei 200—202° schmilzt.

Der Ausgangsstoff wird wie folgt hergestellt: Ein Gemisch von 9,4 g Phthalimidkalium, 31,35 g p-Chlor-o-nitro-benzylchlorid und 75 ml Dimethylformamid wird 3 Stunden unter Rückfluss gekocht und in 180 ml Eiswasser unter Rühren gegossen. Nach 30 Minuten wird das Gemisch filtriert und der Rückstand mit Wasser gewaschen. Man erhält das N-p-Chlor-o-nitrobenzyl-phthalimid, welches bei 190—194° schmilzt.

Ein Gemisch von 29,6 g der letztgenannten Verbindung, 5,5 g Hydrazin-hydrat und 300 ml Aethanol wird 4 Stunden unter Rückfluss gekocht und dann mit 21 ml konz. Chlorwasserstoffsäure versetzt. Nach 30 Minuten wird das Gemisch auf Zimmertemperatur gekühlt, filtriert und der Rückstand mit Wasser gewaschen. Das Filtrat wird konzentriert, das wässerige Konzentrat filtriert und das Filtrat mit 3-normaler wässeriger Natriumhydroxydlösung basisch gemacht. Man extrahiert es mit Diäthyläther, trocknet den Extrakt und dampft ihn ein. Man erhält das p-Chlor-o-nitro-benzylamin.

Eine Lösung vom 42,8 g der letztgenannten Verbindung in 400 ml Eisessig wird mit 30,4 g 2,5-Dimethoxytetrahydrofuran versetzt und das Gemisch eine Stunde unter Rückfluss gekocht. Das Gemisch wird eingedampft, der Rückstand in Eiswasser gegossen und das Gemisch mit Essigsäureäthylester extrahiert. Der Extrakt wird mit gesättigter wässeriger Natriumhydrogencarbonatlösung gewaschen, getrocknet und eingedampft. Der Rückstand wird in Diäthyläther aufgenommen, die Lösung mit Aktivkohle entfärbt, filtriert und eingedampft. Man erhält das 1-(p-Chlor-o-nitro-benzyl)-pyrrol.

Ein Gemisch von 4,26 g der letztgenannten Verbindung, 20 ml Diäthyläther und 1,46 g Chloracetonitril wird in einem Eis-Salzbad gekühlt und unter Rühren mit Chlorwasserstoffgas gesättigt.

Das gesättigte Gemisch wird bei Zimmertemperatur über Nacht gerührt, filtriert und der Rückstand in 50 ml Wasser suspendiert. Die Suspension wird dreimal mit 50 ml Essigsäureäthylester extrahiert. Der Extrakt wird getrocknet und eingedampft. Man erhält das 1-(p-Chlor-o-nitrobenzyl)-2-chloracetyl-pyrrol.

Eine Suspension von 56,5 g der letztgenannten Verbindung in 960 ml Aethanol wird mit 21,8 g N-Methyl-benzylamin und 18,2 g Triäthylamin versetzt und das Gemisch 6 Stunden unter Rückfluss gekocht. Das Reaktionsgemisch wird eingedampft, der Rückstand in Chloroform aufgenommen, die Lösung mit gesättigter wässeriger Natriumcarbonatlösung gewaschen, getrocknet, filtriert und eingedampft. Der Rückstand wird in Diäthyläther gelöst, die Lösung filtriert, das Filtrat eingedampft und der Rückstand mit Methanol trituriert. Man erhält das 1-(p-Chlor-o-nitrobenzyl)-2-(N-methyl-N-benzyl-amino-acetyl)-pyrrol, welches bei 90—93° schmilzt.

Eine Lösung von 1 g der letztgenannten Verbindung in 30 ml Benzol wird mit 0,545 g Chlorameisensäure-äthylester versetzt und das Gemisch 4 Stunden unter Rückfluss gekocht. Das Reaktionsgemisch wird mit Diäthyläther verdünnt, mit 1-normaler Chlorwasserstoffsäure und gesättigter wässeriger Natriumchloridlösung gewaschen, getrocknet und eingedampft. Man erhält das 1-(p-Chlor-o-nitrobenzyl)-2-(N-methyl-N-carbäthoxyamino-acetyl)-pyrrol.

Eine Lösung von 1,43 g der letztgenannten Verbindung in 20 ml Tetrahydrofuran wird mit 15 ml Titan-trichlorid tropfenweise versetzt und das Gemisch über Nacht bei Zimmertemperatur gerührt. Das Reaktionsgemisch wird mit 60 ml einer 10%-igen wässerigen Ammoniaklösung basisch gemacht, filtriert und der Rückstand mit Methylenchlorid gewaschen. Die organische Phase wird abgetrennt, mit gesättigter wässeriger Natriumchloridlösung gewaschen, getrocknet und eingedampft. Man erhält das 8-Chlor-11-(N-methyl-N-carbäthoxyaminomethyl-5H-pyrrolo[2,1-c][1,4]benzodiazepin.

Eine Lösung von 430 mg der letztgenannten Verbindung in 10 ml Aethanol wird mit 760 mg Natriumborhydrid versetzt und das Gemisch über Nacht bei Zimmertemperatur gerührt. Das Reaktionsgemisch wird mit 6-normaler Chlorwasserstoffsäure angesäuert, mit Methylenchlorid verdünnt, mit Wasser und gesättigter wässeriger Natriumchloridlösung gewaschen, getrocknet und eingedampft. Der Rückstand wird auf Silicagel chromatographiert und mit Essigsäureäthylester-Methylenchlorid (1:19) eluiert. Man erhält das 8-Chlor-11-(N-methyl-N-carbäthoxyamino-methyl)-10,11-dihydro-5H-pyrrolo-[2,1-c][1,4]benzodiazepin.

Ein Gemisch von 200 mg der letztgenannten Verbindung, 10 ml Aethanol und 3 ml 20%-iger wässeriger Natriumhydroxydlösung wird 3 Tage unter Rückfluss gekocht und eingedampft. Der Rückstand wird in Wasser aufgenommen, das Gemisch mit Methylenchlorid extrahiert, der Extrakt getrocknet und eingedampft. Man erhält das 8-Chlor-11-(N-methylamino-methyl)-10,11-dihydro-5H-pyrrolo[2,1-c][1,4]benzodiazepin.

Ein Gemisch von 960 mg der letztgenannten Verbindung und 600 mg Oxalsäure-diäthylester wird langsam innerhalb von 45 Minuten auf 140° und innerhalb von 15 Minuten auf 180° erhitzt. Das Reaktionsgemisch wird 30 Minuten bei der letztgenannten Temperatur gehalten, abgekühlt, eingedampft, der Rückstand mit Diäthyläther gewaschen und mit Essigsäureäthylester trituriert. Man erhält das 7-Chlor-2-methyl-3,4-dioxo-1,3,4,14b-tetrahydro-2H,10H-pyrazino[1,2-a]pyrrolo[2,1-c][1,4]benzodiazepin, welches bei 203° schmilzt.

Beispiel 3

Eine Lösung von 1,9 g 2-Methyl-1,3,4,14b-tetrahydro-2H,10H-pyrazino[1,2-a]pyrrolo[2,1-c][1,4]benzodiazepin in 50 ml Benzol wird mit 2,14 g Chlorameisensäureäthylester versetzt und das Gemisch 3 Tage unter Rückfluss gekockt. Das Reaktionsgemisch wird mit Diäthyläther verdünnt, mit 1-normaler Chlorwasserstoffsäure und mit gesättigter wässeriger Lösung von Natriumchlorid und Natriumhydrogencarbonat gewaschen, getrocknet und eingedampft. Man erhält das 2-Carbäthoxy-1,3,4,14b-tetrahydro-2H,10H-pyrazino[1,2-a]pyrrolo[2,1-c][1,4]benzodiazepin, welches in MMR-Spektrum Banden bei 8,55(t), 6,60(m), 5,65(q) und 3,90(d) ppm und im Massenspektrum ein Molekularion von 311 zeigt.

Beispiel 4

Ein Gemisch von 930 mg 2-Carbäthoxy-1,3,4,14b-tetrahydro-2H,10H-pyrazino[1,2-a] pyrrolo[2,1-c] [1,4]benzodiazepin, 40 ml Aethanol und 20 ml einer 20%-igen wässerigen Kaliumhydroxydlösung wird 2 Tage unter Rückfluss gekocht. Nach Abkühlen wird das Reaktionsgemisch mit Essigsäureäthylester verdünnt, mit Wasser gewaschen, getrocknet und eingedampft. Der Rückstand wird in Diäthyläther aufgenommen und die Lösung mit 0,55 g Maleinsäure in einer minimalen Menge Aceton versetzt. Der erhaltene Niederschlag wird abgetrennt und mit Diäthyläther gewaschen. Man erhält das 1,3,4,14b-Tetrahydro-2H,10H-pyrazino[1,2-a]pyrrolo[2,1-c][1,4]benzodiazepin-maleat, welches bei 173—175° schmilzt.

In analoger Weise wird auch das 7-Chlor-1,3,4,14b-tetrahydro-2H,10H-pyrazino[1,2-a]-pyrrolo[2,1-c][1,4]benzodiazepin-maleat (durch Hydrolyse mit 20%-iger wässeriger Natriumhydroxydlösung) erhalten, welches bei 184—186° schimlzt.

Beispiel 5

Eine Lösung von 4,8 g 1,3,4,14b-Tetrahydro-2H,10H-pyrazino[1,2-a]pyrrolo[2,1-c][1,4]-benzodiazepin in 20 ml Dimethylformamid und 2,44 g Triäthylamin wird mit 2,91 g Allylbromid unter Rühren, tropfenweise versetzt. Nach einer Stunde wird das Reaktionsgemisch mit Diäthylather verdünnt, mit Wasser und gesättigter wässeriger Natriumchloridlösung gewaschen und die wässerige Phase mit Diäthyläther extrahiert. Die vereinigten organischen Lösungen werden mit gesättigter wässeriger Natriumhydrogencarbonatlösung gewaschen, getrocknet und der Rückstand wird mit Diäthyläther trituriert. Man erhält das 2-Allyl-1,3,4,14b-tetrahydro-2H,10H-pyrazino[1,2-a]pyrrolo-[2,1-c][1,4]benzodiazepin, welches bei 130—132° schmilzt.

In analoger Weise werden auch das a) 2-(3-Methyl-2-butenyl)-; b) 2-Propargyl-; c) 2-Cyclopropylmethyl-; d) 2-Carbomethoxymethyl und e) 2-Phenyläthyl-1,3,4,14b-tetrahydro-2H,10H-pyrazino[1,2-a]pyrrolo[2,1-c][1,4]benzodiazepin ausgehend von äquivalenten Mengen entsprechender Bromide hergestellt. Die Verbindungen a) bis d) der Erfindung werden in ihre unten genannten Säureadditionssalze, gemäss den Beispielen 1 und 2 übergeführt und aus den angegebenen Lösungsmitteln umkristallisiert. Die Salze schmelzen wie folgt: a) Maleat 152—153° (nach Umkristallisation aus Isopropanol); b) Fumarat, 138—140° (Aethanol); c) Maleat, 189—190° (Isopropanol); d) Maleat, 162—164° (Isopropanol-Diäthyläther) und die freie Base e) schmilzt bei 132—134° nach Umkristallisation aus Isopropanol.

Beispiel 6

Ein Gemisch von 1,5 g 2-Carbomethoxymethyl-1,3,4,14b-tetrahydro-2H,10H-pyrazino[1,2-a]-pyrrolo[2,1-c][1,4]benzodiazepin und 10 ml 1-normaler wässeriger Natriumhydroxydlösung wird 45 Minuten unter Rückfluss gekocht, bis eine homogene Lösung entsteht. Die Lösung lässt man auf Zimmertemperatur abkühlen. Der erhaltene Niederschlag wird abgetrennt und mit Diäthyläther gewaschen. Man erhält das Natriumsalz des 2-Carboxymethyl-1,3,4,14b-tetrahydro-2H,10H-pyrazino-[1,2-a]pyrrolo[2,1-c][1,4]benzodiazepins, welches bei 249—250° schmilzt.

Beispiel 7

Eine Lösung von 300 mg 2-Carbomethoxymethyl-1,3,4,14b-tetrahydro-2H,10H-pyrazino[1,2-a]-pyrrolo[2,1-c] [1,4]benzodiazepin in 5 ml Methanol wird auf ungefähr —70° gekühlt und man leitet 2 Stunden Ammoniakgas in die Lösung bei der genannten Temperatur ein. Das Gemisch wird über Nacht bei Zimmertemperatur gerührt, dann wieder mit Ammoniak gesättigt und 2 Tage bei Zimmertemperatur stehen gelassen. Das Reaktionsgemisch wird eingedampft und der Rückstand mit Diäthyläther trituriert. Man erhält das 2-Carbamoylmethyl-1,3,4,14b-tetrahydro-2H,10H-pyrazino[1,2-a]pyrrolo[2,1-c][1,4]benzodiazepin, welches bei 198—200° schmilzt.

Beispiel 8

Eine Lösung von 780 mg 2-Carbomethoxymethyl-1,3,4,14b-tetrahydro-2H,10H-pyrazino[1,2-a]pyrrolo[2,1-c][1,4]benzodiazepin in 20 ml Tetrahydrofuran wird mit 150 mg Lithiumaluminiumhydrid versetzt und das Gemisch 2 Tage bei Zimmertemperatur gerührt. Das Reaktionsgemisch wird dann mit 0,15 ml Wasser, 0,15 ml einer 15%-igen wässerigen Natriumhydroxydlösung und mit 0,15 ml Wasser in dieser Reihenfolge versetzt, filtriert und das Filtrat eingedampft. Man erhält das 2-(2-Hydroxy - äthyl) - 1,3,4,14b - tetrahydro - 2H,10H - pyrazino[1,2-a]pyrrolo[2,1-c][1,4]benzodiazepin, welches in sein Mono-fumarat umgewandelt wird. Es schmilzt bei 187—189° unter Zersetzung.

Beispiel 9

Ein Gemisch von 100 mg 1,3,4,14b-Tetrahydro-2H,10H-pyrazino[1,2-a]pyrrolo[2,1-c][1,4]-benzodiazepin, 1 ml 1-normaler Chlorwasserstoffsäure und 10 ml Wasser wird zuerst mit einer Lösung von 54,5 mg Natriumcyanat in 2 ml Wasser und dann mit 2 ml Tetrahydrofuran unter Rühren versetzt. Das Reaktionsgemisch wird über Nacht bei Zimmertemperatur gerührt, dann mit Methylenchlorid extrahiert, der Extrakt mit gesättigter wässeriger Natriumcarbonatlösung gewaschen, getrocknet und eingedampft. Man erhält das 2-Carbamoyl-1,3,4,14b-tetrahydro-2H,10H-pyrazino[1,2-a]pyrrolo[2,1-c][1,4]benzodiazepin, welches bei 175—177° schmilzt.

Beispiel 10

Eine Lösung von 1,16 g 2-Allyl-1,3,4,14b-tetrahydro-2H,10H-pyrazino[1,2-a]pyrrolo[2,1-c][1,4]benzodiazepin in 25 ml Aethanol wird über 40 mg Platinoxyd 2 1/2 Stunden bei Zimmertemperatur und atmosphärischem Druck hydriert. Das Reaktionsgemisch wird filtriert, das Filtrat eingedampft, der Rückstand in Isopropanol aufgenommen und die Lösung mit Maleinsäure angesäuert. Man erhält das 2-n-Propyl-1,3,4,14b-tetrahydro-2H,10H-pyrazino[1,2-a]pyrrolo[2,1-c][1,4]benzodiazepin-maleat, welches bei 157—159° schmilzt.

In analoger Weise wird auch das 2-(3-Methyl-butyl)-1,3,4,14b-tetrahydro-2H,10H-pyrazino[1,2-a]pyrrolo[2,1-c][1,4]benzodiazepin-maleat hergestellt. Es schmilzt bei 146—147°.

Beispiel 11

Eine Lösung von 500 mg 1,3,4,14b-Tetrahydro-2H,10H-pyrazino[1,2-a]pyrrolo[2,1-c][1,4]-benzodiazepin in 7,5 ml Dimethylformamid und 350 mg Triäthylamin wird mit 470 mg $\alpha$-Brom-p-chloracetophenon versetzt und das Gemisch bei Zimmertemperatur 2 Stunden gemischt. Das Reaktionsgemisch wird mit 60 ml Diäthyläther verdünnt, mit Wasser gewaschen, getrocknet und eingedampft. Der Rückstand wird in 15 ml Chloroform aufgenommen und die Lösung wieder eingedampft. Man erhält das 2-(p-Chlorbenzoylmethyl)-1,3,4,14b-tetrahydro-2H,10H-pyrazino[1,2-a]-pyrrolo[2,1-c] [1,4]benzodiazepin, welches bei 71—74° schmilzt.

Beispiel 12

Gemäss den in den Beispielen 1 und 2 illustrierten Methoden wird das 12-Formyl-2-methyl-3,4-dioxo-1,3,4,14b-tetrahydro-2H,10H-pyrazino[1,2-a]pyrrolo[2,1-c][1,4]benzodiazepin mit Diboran zum 2,12 - Dimethyl - 1,3,4,14b - tetrahydro - 2H,10H - pyrazino[1,2-a]pyrrolo[2,1-c][1,4]benzodiazepin reduziert. Sein Mono-maleat schmilzt bei 173—175°.

Der Ausgangsstoff wird wie folgt hergestellt: Ein Gemisch von 170 mg Phosphoroxychlorid und 100 ml Dimethylformamid wird bei Zimmertemperatur 30 Minuten gerührt und dann mit einer Lösung von 281 mg 2-Methyl-3,4-dioxo-1,3,4,14b-tetrahydro-2H,10H-pyrazino[1,2-a]pyrrolo[2,1-c][1,4]-benzodiazepin in 5 ml Methylenchlorid tropfenweise versetzt. Nach 30 Minuten wird das Gemisch weitere 30 Minuten unter Rückfluss gekocht und auf Zimmertemperatur abgekühlt. Das Reaktionsgemisch wird mit 1,5 g Natriumacetat in 5 ml Wasser versetzt, 30 Minuten gerührt und die organische Schicht abgetrennt. Sie wird mit Wasser und mit 5%-iger wässeriger Natriumhydrogencarbonatlösung gewaschen, getrocknet, eingedampft und der Rückstand mit Diäthyläther trituriert. Man erhält das 12-Formyl - 2 - methyl - 3,4 - dioxo - 1,3,4,14b - tetrahydro - 2H,10H - pyrazino[1,2-a]pyrrolo[2,1-c][1,4]-benzodiazepin, welches bei 300—302° schmilzt.

Beispiel 13

Ein Gemisch von 500 mg 2-Methyl-1,3,4,14b-tetrahydro-2H,10H-pyrazino[1,2-a]pyrrolo[2,1-c][1,4]benzodiazepin, 10 ml Methylenchlorid und 0,5 ml Methyljodid wird bei Zimmertemperatur eine Stunde gerührt. Das Reaktionsgemisch wird filtriert und der Rückstand mit Methylenchlorid gewaschen. Man erhält das 2,2-Dimethyl-1,3,4,14b-tetrahydro-2H,10H-pyrazino[1,2-a]pyrrolo[2,1-c][1,4]benzodiazepiniumjodid, welches, unter Zersetzung bei 258—260° schmilzt.

Beispiel 14

Eine Lösung von 300 mg 2-Methyl-1,3,4,14b-tetrahydro-2H,10H-pyrazino[1,2-a]pyrrolo[2,1-c][1,4]benzodiazepin in 10 ml methylenchlorid wird mit 290 mg m-Chlorperbenzoesäure in 10 ml Methylenchlorid versetzt und das Gemisch bei Zimmertemperatur 5 Stunden gerührt. Das Reaktionsgemisch wird mit 5 ml 10%-iger wässeriger Natriumsulfitlösung ausgeschüttelt, mit gesättigter wässeriger Natriumhydrogencarbonatlösung gewaschen, getrocknet und eingedampft. Man erhält das 2 - Methyl - 1,3,4,14b - tetrahydro - 2H,10H - pyrazino[1,2-a]pyrrolo[2,1-c][1,4]benzodiazepin - 2-N - oxid, welches bei 143—145° schmilzt.

Beispiel 15

Eine Lösung von 140 mg 2-methyl-1,4-dioxo-1,3,4,14b-tetrahydro-2H,10H-pyrazino[1,2-a]-pyrrolo[2,1-c][1,4]benzodiazepin in 5 ml Tetrahydrofuran wird unter Rühren und Kühlen mit Eis mit 2 ml 1-molarem Diboran in Tetrahydrofuran versetzt. Das Reaktionsgemisch wird bei Zimmertemperatur über nacht gerührt und dann 4 Stunden unter Rückfluss gekocht. Nach Abkühlen auf Zimmertemperatur wird es mit 0,5 ml Eisessig versetzt, èingedampft und der Rückstand mit 3-normaler wässeriger Natriumhydroxydlösung basisch gestellt. Das Gemisch wird mit Methylenchlorid extrahiert, der Extrakt getrocknet, eingedampft und der Rückstand in Diäthyläther aufgenommen. Die Lösung wird filtriert und das Filtrat eingedampft. Man erhält das 2-Methyl-1,3,4,14b-tetrahydro-2H,10H-pyrazino-[1,2-a]pyrrolo[2,1-c][1,4]benzodiazepin, welches mit dem Produkt des Beispiels 1 identisch ist. Sein Mono-maleat schmilzt bei 176—178°.

Der Ausgangsstoff wird wie folgt hergestellt: Eine Lösung von 16 g Oxalylchlorid in 150 ml Diäthyläther wird unter Rühren und Kühlen auf —50° (festes Kohlendioxyd-Aceton-Bad) mit einer Lösung von 7,36 g Pyrrol in 30 ml Diäthyläther derart versetzt, dass die innere Temperatur des Reaktionsgemisches bei —50° bleibt. nach Abschluss der Zugabe wird es eine Stunde gerührt und dann langsam in die Lösung von 27,5 g Sarkosin-äthylester in 150 ml Diäthyläther gegossen. Der erhaltene Niederschlag wird filtriert, mit Methylenchlorid sorgfältig extrahiert und der Extrakt eingedampft. Man erhält den N-(2-Pyrrylglyoxy)-sarkosinäthylester, der bei 114° schmilzt.

Eine Lösung von 5,5 g der letztgenannten Verbindung in 30 ml Dimethylformamid wird mit 1,07 g einer 50%-igen Natriumhydridsuspension in Mineralöl und mit 20 ml Dimethylformamid versetzt. Das Gemisch wird eine Stunde auf 60—70° erhitzt, auf Zimmertemperatur gekühlt und mit einer Lösung von 5 g o-Nitrobenzylbromid in 20 ml Dimethylformamid versetzt. Das Reaktionsgemisch wird eine Stunde bei 45—50° gerührt, auf Zimmertemperatur gekühlt, mit Wasser verdünnt und mit Essigsäureäthylester extrahiert. Der Extrakt wird getrocknet, eingedampft und der Rückstand mit

Diäthyläther trituriert. Man erhält den N-(1-o-Nitrobenzyl-2-pyrrylglyoxyl)-sarkosin-äthylester, welcher bei 105—108° schmilzt.

Eine Lösung von 3 g der letztgenannten Verbindung in 30 ml Essigsäureäthylester wird über 100 mg Platinoxid bei 3 Atmosphären bis zur Aufnahme von 3 Moläquivalenten Wasserstoff hydriert. Das Gemisch wird mit 1,5 ml Eisessig versetzt und die Hydrierung bei 3 Atmosphären und 40° abgeschlossen. Das Reaktionsgemisch wird filtriert, das Filtrat eingedampft, der Rückstand in Methylenchlorid aufgenommen und durch Chromatographie auf Silicagel gereinigt. Man eluiert mit 10%-igem Methanol-Methylenchlorid und erhält den N-(1,2-Dihydro-5H-pyrrolo[2,1-c][1,4]benzodiazepin-2-yl-carbonyl)-sarkosin-äthylester, welcher bei 147—148° schmilzt.

Eine Suspension von 100 mg der letztgenannten Verbindung in 10 ml Toluol wird mit 20 mg Natriummethoxid versetzt und das Gemisch 1 Stunde unter Rückfluss gekocht. Das Reaktionsgemisch wird abgekühlt, filtriert und eingedampft. Man erhält das 2-Methyl-1,4-dioxo-1,3,4,14b-tetrahydro-2H,10H-pyrazino[1,2-a]pyrrolo[2,1-c][1,4]benzodiazepin, welches bei 165—167° schmilzt.

## Beispiel 16

Eine heisse Lösung von 10 g 2-Methyl-1,3,4,14b-tetrahydro-2H,10H-pyrazino[1,2-a]pyrrolo-[2,1-c][1,4]benzodiazepin (Beispiel 1) in 300 ml Isopropanol wird mit einer heissen Lösung von 3 g d-Weinsäure in 50 ml Isopropanol versetzt. Das Gemisch kühlt sich über Nacht auf Zimmertemperatur ab. Der erhaltene Niederschlag wird abfiltriert und aus wässerigem Aethanol so lange umkristallisiert (dreimal), bis die optische Drehung der freigesetzten Base konstant, nämlich $[\alpha]_D^{25} = +344,26°$ ist (c = 1 in Methanol). Die Verbindung wird in ihr Mono-maleat umgewandelt, welches bei 190—191° unter Zersetzung schmilzt.

Unter Verwendung der l-Weinsäure erhält man den entsprechenden l-Antipoden, $[\alpha]_D^{25} = -358,89°$, dessen Mono-maleat bei 188—189° unter Zersetzung schmilzt.

## Beispiel 17

Eine Lösung von 800 mg 2-Methyl-5-oxo1,2,3,4,5,15b-hexahydro-11H-[1,4]diazepino[1,2-a]-pyrrolo[2,1-c][1,4]benzodiazepin in 50 ml Tetrahydrofuran wird mit 6,2 ml 1-molarem Diboran in Tetrahydrofuran versetzt. Das Gemisch wird 2 Stunden unter Rückfluss gekocht, abgekühlt und über Nacht bei Zimmertemperatur gerührt. Das Reaktionsgemisch wird mit 2 ml Eisessig versetzt, eingedampft und der Rückstand mit 3-normaler wässeriger Natriumhydroxydlösung basisch gemacht. Das erhaltene Gemisch wird mit Methylenchlorid extrahiert, der Extrakt getrocknet, eingedampft und der Rückstand in einer minimalen Menge Methylenchlorid gelöst. Die Lösung wird auf Silicagel chromatographiert und mit 10%-igem Methanol-Methylenchlorid eluiert. Man erhält das 2-Methyl-1,2,3,4,5,15b-hexahydro-11H-[1,4]diazepino[1,2-a]pyrrolo[2,1-c][1,4]benzodiazepin der Formel I, in welcher $R_1=R_3=R_4=R_5=H$, $R_2$ = Methyl, Ph = 1,2-Phenylen und n = 3. Das Produkt wird in sein Monofumarat umgewandelt, welches bei 174—175° schmilzt.

Der Ausgangsstoff wird wie folgt hergestellt: Eine Lösung von 1,5 g 11-(N-Methylaminomethyl)-10,11-dihydro-5H-pyrrolo[2,1-c][1,4]benzodiazepin (Beispiel 1) in 7,5 ml Acrylsäure-methylester wird 24 Stunden bei Zimmertemperatur gerührt und das überschüssige Reagens abgedampft. Man erhält dan N-Methyl-N-(1,2-dihydro-5H-pyrrolo[2,1-c][1,4]benzodiazepin-2-yl-methylen)-$\beta$-alanin-methylester als ein Oel, welches auf Silicagel chromatographiert und mit 2%-igem Methanol-Methylenchlorid eluiert wird.

Eine Lösung von 1 g der letztgenannten Verbindung in 20 ml Tetrahydrofuran wird zu einer gerührten Lösung von 475 mg 2,2,6,6-Tetramethyl-piperidin (welches über Calciumhydrid frisch destilliert ist) in 10 ml Tetrahydrofuran gegeben und dann mit 1,37 ml 2,45-molarem n-Butyllithium in Hexan, bei —75° versetzt. Das Gemisch wird bei dieser Temperatur 30 Minuten gerührt, und man lässt es dann auf 0° erwärmen. Das Gemisch wird mit Essigsäure-Wasser (2:1) verdünnt, eingedampft, der Rückstand in Methylenchlorid gelöst, auf Silicagel chromatographiert und mit 5%-igem Methanol-Methylenchlorid eluiert. Man erhält das 2-Methyl-5-oxo-1,2,3,4,5,15b-hexahydro-11H-[1,4]-diazepino[1,2-a]pyrrolo[2,1-c][1,4]benzodiazepin, welches bei 156—158° schmilzt.

## Beispiel 18

Eine Lösung von 2,5 g 2-Methyl-1,3,4,14b-tetrahydro-2H,10H-pyrazino[1,2-a]pyrrolo[2,1-c][1,4]benzodiazepin (Beispiel 1) in 25 ml Tetrahydrofuran wird bei Zimmertemperatur, unter Rühren in einer Stickstoffatmosphäre, mit 4,5 ml 2,45-molarem n-Butyl-lithium in Hexan versetzt. Die schwach gelbe Lösung wird schnell kirschrot und die Temperatur steigt ungefähr um 7°. Das Gemisch wird weitere 45 Minuten gerührt, und dann mit Methyljodid bis zur Entfärbung der Lösung behandelt. Das Reaktionsgemisch wird weitere 30 Minuten gerührt, dann in 100 ml Wasser gegossen und das Produkt mit Diäthyläther extrahiert. Der Extrakt wird nacheinander mit Wasser und gesättigter wässeriger Natriumchloridlösung gewaschen und eingedampft. Das als Rückstand erhaltene Oel wird auf Silicagel chromatographiert und mit 2%-igem Methanol-Methylenchlorid eluiert. Man erhält das 2,10-Dimethyl-1,3,4,14b-tetrahydro-2H,10H-pyrazino[1,2-a]pyrrolo[2,1-c][1,4]benzodiazepin, welches in sein Monomaleat umgewandelt wird. F. 157°. Das gleiche Produkt ist auch gemäss den Verfahren der Beispiele 1

und 15 erhältlich, indem man zur Herstellung des Ausgangsstoffes o-Nitro-$\alpha$-methyl-benzylchlorid oder -bromid verwendet.

Beispiel 19

Eine gerührte Lösung von 1,3 g 1,3,4,14b-Tetrahydro-2H,10H-pyrazino[1,2-a]pyrrolo[2,1-c][1,4]-benzodiazepin (Beispiel 4) und 550 mg Triäthylamin in 15 ml Tetrahydrofuran wird mit 765 mg Benzoylchlorid versetzt. Das Reaktionsgemisch wird über Nacht bei Zimmertemperatur gerührt, dann eingedampft und der Rückstand in 100 ml Wasser aufgenommen. Das Gemisch wird mit Diäthyläther extrahiert, der Extrakt nacheinander mit Wasser und gesättigter wässeriger Natriumchloridlösung gewaschen, getrocknet und eingedampft. Man erhält das 2-Benzoyl-1,3,4,14b-tetrahydro-2H,10H-pyrazino[1,2-a]pyrrolo[2,1-c][1,4]benzodiazepin, welches nach Umkristallisation aus Isopropanol bei 137—139° schmilzt.

Beispiel 20

Eine Lösung von 2,5 g 2-(2-Hydroxyäthyl)-1,3,4,14b-tetrahydro-2H,10H-pyrazino[1,2-a]pyrrolo-[2,1-c][1,4]benzodiazepin (Beispiel 8) und 935 mg Triäthylamin in 72 ml Tetrahydrofuran wird mit 720 mg Acetylchlorid versetzt. Das Gemisch wird 2 Stunden bei Zimmertemperatur gerührt, eingedampft und der Rückstand mit Methylenchlorid extrahiert. Der Extrakt wird nacheinander mit Wasser und gesättigter wässeriger Natriumchloridlösung gewaschen, getrocknet, filtriert und eingedampft. Man erhält das 2-(2-Acetoxyäthyl)-1,3,4,14b-tetrahydro-2H,10H-pyrazino[1,2-a]pyrrolo[2,1-c][1,4]benzodiazepin, welches in sein Mono-fumarat umgewandelt wird. F. 156—158° (unter Zersetzung).

In analoger Weise werden die Mono-fumarate von 2-[2-(n-Hexanoyloxy, n-Decanoyloxy, n-Hexadecanoyloxy und 1-Adamantylcarbonyloxy)-äthyl]-1,3,4,14b-tetrahydro-2H,10H-pyrazino[1,2-a]-pyrrolo[2,1-c][1,4]benzodiazepin hergestellt. Diese Verbindungen schmelzen bei 56—58°, 59—61°, 71—73° bzw. 47—50°.

Beispiel 21

Man gibt portionenweise innerhalb einer Stunde 348 g 2-Methyl-3,4-dioxo-1,3,4,14b-tetrahydro-2H,10H-pyrazino[1,2-a]pyrrolo[2,1-c][1,4]benzodiazepin zu 4500 ml Tetrahydrofuran und 7410 ml 1-molarem Diboran in Tetrahydrofuran unter Kühlen mit Eis auf 18° und Rühren in einer Stickstoffatmosphäre. Das Gemisch wird 24 Stunden unter Rückfluss gekocht, auf 5° gekühlt und zuerst mit 1200 ml Eisessig und dann mit 900 ml Wasser versetzt. Die Lösung wird 24 Stunden unter Rückfluss gekocht, eingedampft und der Rückstand in 10'500 ml Methanol aufgenommen. Die Lösung wird wieder 2 Stunden unter Rückfluss gekocht, eingedampft, der Rückstand in 3000 ml Wasser gelöst und der pH-Wert der Lösung mit 1200 ml 10%-iger wässeriger Natriumhydroxydlösung auf 14 eingestellt. Das Gemisch wird mit Diäthyläther extrahiert, der Extrakt getrocknet, filtriert und eingedampft. Man erhält das 2-Methyl-1,3,4,14b-tetrahydro-2H,10H-pyrazino[1,2-a]pyrrolo[2,1-c][1,4]benzodiazepin, welches bei 98—100° schmilzt.

Man löst 307 g der letztgenannten Verbindung in 1280 ml absolutem Aethanol unter Rückfluss, filtriert die Lösung, kühlt das Filtrat auf 28° ab und versetzt es mit 140,5 g Maleinsäure in 294 ml Aethanol. Der erhaltene Niederschlag wird abgetrennt, mit kaltem Aethanol gewaschen und wieder aus Aethanol umkristallisiert. Man erhält das entsprechende Mono-maleat, welches bei 183—185° schmilzt. Das Produkt ist etwas reiner als dasjenige des Beispiels 1.

Der ausgangsstoff wird wie folgt hergestellt: Ein Gemisch von 3078 g o-Nitro-benzylamin und 2670 g 2,5-Dimethoxy-tetrahydrofuran wird schnell zu 10'000 ml Eisessig unter Rühren bei 86° in einer Stickstoffatmosphäre gegeben. Das Gemisch wird 1 1/4 Stunde bei 95° gerührt, auf 25° gekühlt und mit 30'000 ml Wasser versetzt. Das Reaktionsgemisch wird mit Essigsäureäthylester extrahiert, der Extrakt mit 10%-iger wässeriger Natriumhydroxydlösung und mit 10%-iger wässeriger Natriumchloridlösung gewaschen, filtriert und eingedampft. Man erhält das 1-(o-Nitrobenzyl)-pyrrol.

Ein Gemisch von 3468 g der letztgenannten Verbindung, 8500 ml Tetrahydrofuran und 1290 g Chloracetonitril wird unter Rühren und Kühlen auf 5—35° drei Stunden mit Chlorwasserstoffgas gesättigt. Die gesättigte Suspension wird eine weitere Stunde bei 17° gerührt, filtriert, der Rückstand in 1000 ml Tetrahydrofuran suspendiert und wieder filtriert. Man erhält das 1-o-Nitrobenzyl-2-(1-imino-2-chlor-äthyl)-pyrrol-hydrochlorid, welches unter Zersetzung bei 210—212° schmilzt.

Eine Suspension von 3469 g der letztgenannten Verbindung in 3500 ml Wasser wird eine Stunde bei 80° gerührt, dann auf 25° gekühlt und filtriert. Der Rückstand wird mit Wasser gewaschen und getrocknet. Man löst 6000 g des Rückstandes in 60'000 ml Aethanol unter Kochen und Rückfluss in einer Stickstoffatmosphäre. Die Lösung wird heiss filtriert, durch Abdestillieren von 36'000 ml Aethanol konzentriert und über Nacht auf 25° abgekühlt. Die erhaltene Suspension wird filtriert und der Rückstand getrocknet. Man erhält das 1-o-Nitrobenzyl-2-chloracetyl-pyrrol, welches bei 112—114° schmilzt.

Eine Suspension von 5112 g der letztgenannten Verbindung, 26'400 ml Toluol, 2445 g N-Methyl-benzylamin und 2040 g Triäthylamin wird in einer Stickstoffatmosphäre 6 Stunden bei 93° und über Nacht bei Zimmertemperatur gerührt. Das Reaktionsgemisch wird mit 20'000 ml Wasser versetzt, die wässerige Phase abgetrennt, die organische Phase mit 10%-iger wässeriger Natriumchloridlösung

gewaschen und eingedampft. Der Rückstand wird in 20'000 ml heissem Aethanol gelöst, die Lösung durch Abdestillieren von 2000 ml Aethanol konzentriert und über Nacht bei Zimmertemperatur gerührt. Die erhaltenen Kristalle werden abfiltriert, mit Aethanol gewaschen und getrocknet. Man erhält das 1-(o-Nitrobenzyl)-2-(N-methyl-N-benzylamino-acetyl)-pyrrol, welches bei 103—105° schmilzt.

Eine Lösung von 300 g der letztgenannten Verbindung in 3000 ml Essigsäureäthylester und 250 ml Eisessig wird über 30 g Platinoxyd bei Zimmertemperatur und atmosphärischem Druck bis zur Aufnahme der theoretischen Menge Wasserstoff hydriert. Das Reaktionsgemisch wird filtriert, der Rückstand mit Essigsäureäthylester gewaschen und das Filtrat eingedampft. Der Rückstand wird in Methylenchlorid aufgenommen, die Lösung mit 2,5-normaler wässeriger Natriumhydroxydlösung und mit gesättigter wässeriger Natriumchloridlösung gewaschen, getrocknet und eingedampft. Der Rückstand wird mit Diäthyläther trituriert. Man erhält das 11-(N-Methyl-N-benzylaminomethyl)-10,11-dihydro-5H-pyrrolo[2,1-c][1,4]benzodiazepin, welches bei 151—152° schmilzt.

Eine Lösung von 142,5 g der letztgenannten Verbindung in 2000 ml Toluol und 45 g Triäthylamin wird unter Rühren bei 10—20° innerhalb von 90 Minuten mit 61 g Oxalsäure-hemi-äthylester-chlorid in 850 ml Toluol versetzt. Nach 4 Stunden wird das Gemisch in 750 ml Wasser gegossen, 20 Minuten gerührt und die organische Schicht abgetrennt. Sie wird mit gesättigter wässeriger Natriumhydrogencarbonatlösung und mit gesättigter wässeriger Natriumchloridlösung gewaschen, getrocknet und eingedampft. Der Rückstand wird aus Methanol umkristallisiert. Man erhält das 10-Aethyloxalyl-11-(N-methyl-N-benzylaminomethyl)-10,11-dihydro-5H-pyrrolo[2,1-c][1,4]benzodiazepin, welches bei 99—101° schmilzt. (Bei Verwendung von grösseren Mengen Oxalsäure-hemi-äthylester-chlorid erhält man die entsprechende 3,10-bis-Aethyloxalyl-Verbindung, welche bei 115—116° schmilzt. Diese ist in Isopropanol weniger löslich als die vorher genannte Verbindung und kann aus konzentrierten Lösungen durch Filtrieren abgretrennt werden.)

Eine Lösung von 6,9 g der genannten 10-Aethyloxalyl-Verbindung in 100 ml Aethanol und 27 ml Eisessig wird über 1,65 g eines 5%-igen Palladium-auf-Kohle-Katalysators, bei 2,7 Atmosphären und 40° ungefähr eine Stunde hydriert. Das Gemisch wird filtriert und das Filtrat eingedampft. Man erhält das 2 - Methyl - 3,4 - dioxo - 1,3,4,14b - tetrahydro - 2H,10H - pyrazino[1,2-a]pyrrolo[2,1-c][1,4]-benzodiazepin, welches bei 178—179° schmilzt.

Durch Hydrierung der genannten 3,10-bis-Aethyloxalyl-Verbindung in analoger Weise, erhält man das (2 - Methyl - 3,4 - dioxo - 1,3,4,14b - tetrahydro - 2H,10H - pyrazino[1,2-a]pyrrolo[2,1-c][1,4]-benzodiazepin - 12 - yl) - äthylglykolat.

Beispiel 22

Reduziert man 200 mg des (2-Methyl-3,4-dioxo-1,3,4,14b-tetrahydro-2H,10H-pyrazino[1,2-a]-pyrrolo[2,1-c][1,4]benzodiazepin-12-yl)-äthylglykolats (Beispiel 21) gemäss dem Beispiel 1 oder 2, erhält man das 2-Methyl-12-(2-hydroxyäthyl)-1,3,4,14b-tetrahydro-2H,10H-pyrazino[1,2-a]pyrrolo-[2,1-c][1,4]benzodiazepin-monomaleat, welches bei 153—156° schmilzt.

Beispiel 23

Eine Lösung von 5 g 2-Methyl-1,3,4,14b-tetrahydro-2H,10H-pyrazino[1,2-a]pyrrolo[2,1-c][1,4]-benzodiazepin und 2 g Triäthylamin in 50 ml Toluol wird mit 3,6 g Trichloracetyl-chlorid versetzt. Das Gemisch wird eine Stunde gerührt und dann mit 30 ml Wasser und 50 ml Methylenchlorid versetzt. Die organische Phase wird mit Wasser gewaschen, getrocknet und eingedampft. Man erhält das 2-Methyl-12-trichloracetyl-1,3,4,14b-tetrahydro-2H,10H-pyrazino[1,2-a]pyrrolo[2,1-c][1,4]benzodiazepin.

Man löst 100 mg Natrium in 75 ml Aethanol und versetzt die Lösung mit 5,4 g der vorher genannten 12-Trichloracetyl-Verbindung. Der erhaltene Niederschlag wird abfiltriert, mit Wasser gewaschen und getrocknet. Man erhält das 2-Methyl-12-carbäthoxy-1,3,4,14b-tetrahydro-2H,10H-pyrazino[1,2-a]pyrrolo[2,1-c][1,4]benzodiazepin, welches bei 128—130° schmilzt. Sein Monofumarat-monohydrat schmilzt bei 147—150° unter Zersetzung.

Beispiel 24

Gemäss den in den vorhergehenden Beispielen beschriebenen Methoden (vorzugsweise in den Beispielen 1 und 21) werden die folgenden Verbindungen der Formel II ausgehend von äquivalenten Mengen entsprechender Ausgangsstoffe hergestellt:

| Nr. | $R_6$ | $R_7$ | Salz | F. °C (Zersetzung) |
|---|---|---|---|---|
| 1 | H | 9-$CH_3$ | Maleat | 185–187 |
| 2 | $CH_3$ | 9-$CH_3$ | Maleat | 172–174 |
| 3 | $CH_2{=}CH{-}CH_2$ | 9-$CH_3$ | Maleat | 159–161 |
| 4 | H | 8-$CH_3$ | Maleat | 169–171 |
| 5 | $CH_3$ | 8-$CH_3$ | Maleat | 162–164 |
| 6 | $CH_3$ | 7-F | Maleat | 179–181 |
| 7 | H | 7-$CF_3$ | Maleat | 172–174 |
| 8 | $CH_3$ | 7-$CF_3$ | Maleat | 189–191 |
| 9 | $(CH_2)_3{-}OH$ | H | Fumarat | 201–203 |
| 10 | $(CH_2)_2{-}CO{-}CH_3$ | H | Fumarat | 155–157 |
| 11 | $(CH_2)_{11}{-}CH_3$ | H | Fumarat | 144–148 |

Beispiel 25

Herstellung von 10'000 Tabletten mit einem Gehalt von je 5 mg der aktiven Substanz:

| Bestandteile: | |
|---|---|
| 2-Methyl-1,3,4,14b-tetrahydro-2H,10H-pyrazino[1,2-a]-pyrrolo[2,1-c][1,4]benzodiazepin-monomaleat | 50 g |
| Milchzucker | 1157 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

*Verfahren:* Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zu der siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 26

Herstellung von 10'000 Kapseln mit einem Gehalt von je 10 mg der aktiven Substanz:

| Bestandteile: | |
|---|---|
| 2-methyl-1,3,4,14b-tetrahydro-2H,10H-pyrazino[1,2-a]-pyrrolo[2,1-c][1,4]benzodiazepin-monomaleat | 100 g |
| Milchzucker | 1800 g |
| Talkpulver | 100 g |

*Verfahren:* Sämtliche pulverigen Bestandteile werden mit einem Sieb von 0,6 mm Maschenweite gesiebt. Dann wird der Wirkstoff zuerst mit Talk und darauffolgend mit dem Milchzucker in einem geeigneten Mischer homogenisiert. Kapseln Nr. 3 werden mit je 200 mg des Gemisches mit einer Füllmaschine gefüllt.

In analoger Weise werden Tabletten oder Kapseln, welche eine andere Verbindung der Erfindung, z.B. eine solche der vorhergehenden Beispiele enthalten, hergestellt.

**Patentansprüche**

1. 1,3,4,14b-Tetrahydro-2H,10H-pyrazino[1,2-a]pyrrolo[2,1-c][1,4]benzodiazepine der allgemeinen Formel I

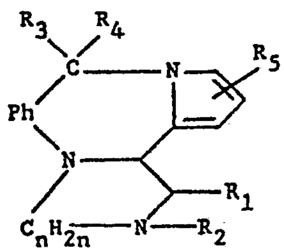

(I)

worin jedes der Symbole $R_1$, $R_3$ und $R_4$ Wasserstoff oder Niederalkyl bedeutet, $R_2$ für Wasserstoff, Niederalkyl, Höheralkyl, Niederalkenyl, Niederalkynyl, Cycloalkyl oder Cycloalkenyl mit jeweils 3 bis 7 Ringgliedern oder (Cycloalkyl, Hydroxy, Amino, Mono- oder Di-niederalkylamino, Carboxy, niederes Carbalkoxy, Carbamoyl, Mono- oder Di-niederalkyl-carbamoyl, HPh, Niederalkanoyl oder HPhCO)-niederalkyl steht, Ph 1,2-Phenylen bedeutet, welches durch höchstens 2 Substituenten der Gruppe bestehend aus Niederalkyl, Niederalkoxy, Niederalkylthio, Halogen und Trifluormethyl substituiert sein kann, $C_nH_{2n}$ für Niederalkylen steht, welches die zwei Stickstoffatome durch 2 oder 3 Kohlenstoffatome trennt, und $R_5$ Wasserstoff, Niederalkyl, Carboxy, niederes Carbalkoxy oder (Hydroxy, Amino, Mono- oder Di-niederalkylamino)-niederalkyl bedeutet; die Niederalkoxycarbonyl-, Niederalkanoyl-, Höheralkanoyl-, Adamantoyl-, Carbamoyl-, Mono- oder Di-niederalkylcarbamoyl-, Cycloalkyl-carbonyl-, mit 3 bis 7 Ringgliedern, oder HPhCO-Derivate, die 2-N-Oxide, die 2-Niederalkyl- oder 2-HPh-Niederalkyl-quaternären Ammoniumderivate und ihre Salze.

2. Verbindungen der im Anspruch 1 angegebenen Formel I, worin jedes der Symbole $R_1$, $R_3$ und $R_4$ Wasserstoff bedeutet, $R_2$ für Wasserstoff, Niederalkyl, Niederalkenyl, Niederalkynyl, Hydroxy-niederalkyl, Cycloalkyl oder Cycloalkyl-niederalkyl mit jeweils 3 bis 7 Ringkohlenstoffatomen steht, Ph 1,2-Phenylen, (Niederalkyl)-1,2-phenylen, (Niederalkoxy)-1,2-phenylen, (Niederalkylthio)-1,2-phenylen, (Halogen)-1,2-phenylen oder (Trifluormethyl)-1,2-phenylen bedeutet, n eine ganze Zahl von 2 oder 3 ist, und $R_5$ Wasserstoff, Niederalkyl oder Hydroxy-niederalkyl bedeutet, ihre Niederalkanoyl-, Adamantoyl-, Carbamoyl-, Mono- oder Di-niederalkylcarbamoyl oder HPhCO-Derivate, 2-N-Oxide, 2-Niederalkyl- oder 2-HPh-Niederalkyl quaternären Derivate und ihre therapeutisch verwendbaren Säureadditionssalze.

3. Verbindungen der allgemeinen Formel II,

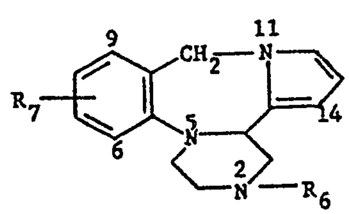

(II)

worin $R_6$ Wasserstoff, Niederalkyl, Niederalkenyl, Niederalkynyl, Hydroxy-niederalkyl oder Cycloalkyl-niederalkyl mit 3 bis 7 Ringgliedern bedeutet, und $R_7$ für Wasserstoff, Halogen oder Trifluormethyl steht, und ihre therapeutisch verwendbaren Säureadditionssalze.

4. Verbindungen der im Anspruch 3 gezeigten Formel II, worin $R_6$ Alkyl, Alkenyl, Alkynyl oder Hydroxyalkyl mit jeweils bis 4 Kohlenstoffatomen oder Cyclopropylmethyl bedeutet, $R_7$ für Fluor oder Chlor steht, und ihre therapeutisch verwendbaren Säureadditionssalze.

5. Verbindungen der im Anspruch 3 gezeigten Formel II, worin alle Symbole die im Anspruch 4 angegebenen Bedeutungen haben, wobei $R_7$ in 7-Stellung steht.

6. 2-Methyl-1,3,4,14b-tetrahydro-2H,10H-pyrazino[1,2-a]pyrrolo[2,1-c][1,4]benzodiazepin, seine d- und l-Antipoden, ihre 2-N-Oxide und ihre therapeutisch verwendbaren Säureadditionssalze.

7. 2-(2-Hydroxy-äthyl)-1,3,4,14b-Tetrahydro-2H,10H-pyrazino[1,2-a]pyrrolo[2,1-c][1,4]benzodiazepin und seine therapeutisch verwendbaren Säureadditionssalze.

8. Pharmazeutische Präparate enthaltend eine Verbindung gemäss einem der Ansprüche 1 bis 7 zusammen mit einem pharmazeutischen Trägermaterial.

9. Verbindungen der im Anspruch 1 angegebenen Formel I zur Anwendung in einem Verfahren zur therapeutischen Behandlung des menschlichen oder tierischen Körpers.

10. Die Verbindungen der Ansprüche 1 bis 7 zur Verwendung als Pharmazeutika.

11. Verbindungen der im Anspruch 1 angegebenen Formel I zur Verwendung als Antidepressiva, Analgetika und Antihistaminika.

12. Verfahren zur Herstellung von neuen 1,3,4,14b-Tetrahydro-2H,10H-pyrazino[1,2-a]pyrrolo-[2,1-c][1,4]benzodiazepin gemäss der Allegemeinen Formel I, des Anspruches 1 dadurch gekennzeichnet, dass man eine Verbindung der allgemeinen Formel III

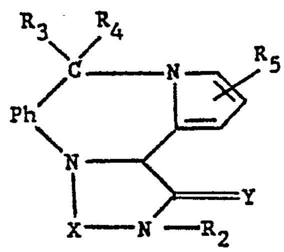

(III),

worin X Niederalkylen, Mono- oder Dioxo-niederalkylen bedeutet, welches die zwei Stickstoffatome durch 2 oder 3 Kohlenstoffatome trennt und worin Oxo zu dem mit dem Stickstoffatom benachbarten Kohlenstoffatom gebunden ist, und Y für Oxo, zwei Wasserstoffatome oder Wasserstoff und Niederalkyl steht, mit der Massgabe, dass mindestens eine Oxogruppe in X oder Y vorhanden ist, reduziert, und, wenn eine Verbindung erwünscht ist, worin $R_2$ Niederalkyl, Höheralkyl, Niederalkenyl, Niederalkynyl, Cycloalkyl, Cycloalkyl-niederalkyl, Cycloalkenyl, HPh-niederalkyl oder Carbalkoxy-niederalkyl bedeutet, in eine Verbindung der Formel I, worin $R_2$ Wasserstoff bedeutet, einen der genannten Reste einführt, und/oder, wenn eine Verbindung erwünscht ist, worin $R_2$ Carboxy-niederalkyl bedeutet, eine Verbindung der Formel I, worin $R_2$ Carbalkoxy-niederalkyl bedeutet, hydrolysiert, und/oder, wenn eine Verbindung erwünscht ist, worin $R_2$ Carbamoyl-niederalkyl, (Mono- oder Di-niederalkyl-carbamoyl)-niederalkyl bedeutet, in einem Endprodukt, worin $R_2$ Carbalkoxy-niederalkyl bedeutet, diese Gruppe in einen der genannten Reste umwandelt, und/oder, wenn eine Verbindung erwünscht ist, worin $R_2$ Niederalkanoyl-niederalkyl oder HPhCO-niederalkyl ist, in ein Endprodukt, worin $R_2$ Wasserstoff bedeutet, einen der genannten Reste einführt, und/oder, wenn eine Verbindung erwünscht ist, worin $R_2$ (Mono- oder Di-niederalkylcarbamoyl)-niederalkyl ist, ein Endprodukt, worin $R_2$ Carbamoyl-niederalkyl oder (Mono-niederalkylcarbamoyl)-niederalkyl ist, alkyliert, und/oder, wenn eine Verbindung erwünscht ist, die in 2-Stellung einen oben genannten Acylrest aufweist, ein Produkt, worin $R_2$ Wasserstoff bedeutet, acyliert, und/oder, wenn eine Verbindung erwünscht ist, worin $R_2$ Niederalkylcarbonyl bedeutet, in einem Endprodukt, worin $R_2$ Niederalkyl, Niederalkenyl oder $-CH_2-$HPh bedeutet, diese Gruppen in eine Niederalkoxycarbonylgruppe überführt, und/oder, wenn eine Verbindung erwünscht ist, worin der Substituent am Stickstoffatom in 2-Stellung und/oder $R_5$ ein Niederalkanoyloxy-niederalkyl oder ein Höheralkanoyloxy-niederalkylrest ist, ein Produkt, mit einer oder zwei Hydroxy-niederalkylgruppen verestert, und/oder, wenn eine Verbindung erwünscht ist, worin $R_3$ und/oder $R_4$ Niederalkyl bedeutet, in einer erhaltenen Verbindung, worin $R_3$ und/oder $R_4$ Wasserstoff bedeutet, Niederalkyl einführt, und/oder, wenn eine Verbindung erwünscht ist, worin $R_5$ in 12-Stellung ein Acylrest ist, ein Endprodukt in dieser Stellung acyliert, und/oder, wenn eine Verbindung erwünscht ist, worin $R_5$ in 12-Stellung eine Carboxygruppe ist, ein Endprodukt, worin $R_5$ Niederalkoxy-carbonyl bedeutet, hydrolysiert, und, wenn eine quaternäre Verbindung erwünscht ist, ein Produkt, das in 2-Stellung substituiert ist, durch Einführung eines Niederalkyl- oder HPh-niederalkylrestes in 2-Stellung quaternisiert, und/oder, wenn ein N-Oxyd erwünscht ist, ein Produkt, worin das Stickstoffatom in 2-Stellung einen Substituenten aufweist, N-oxydiert, und/oder wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder

wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

## Revendications

1. 1,3,4,14b-Tétrahydro-2H,10H-pyrazino[1,2-a]pyrrolo[2,1-c][1,4]benzodiazépines de formule générale (I)

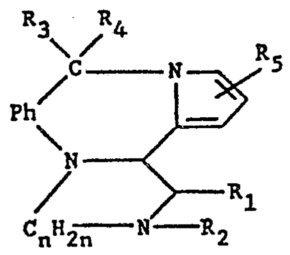

(I)

où chacun des symboles $R_1$, $R_3$ et $R_4$ représente un hydrogène ou un alcoyl inférieur, $R_2$ un hydrogène, un alcoyle inférieur, un alcoyle supérieur, un alcényle inférieur, un alcynyle inférieur, un cycloalcoyle ou un cycloalcényle ayant chacun de 3 à 7 chaînons dans le noyau ou un (cycloalcoyle, hydroxy, amino, mono- ou di-alcoyle inférieur-amino, carboxy, carbalcoxy inférieur, carbamoyle, mono- ou dialcoyle inférieur-carbamoyle, HPh, alcanoyle inférieur ou HPhCO)-alcoyle inférieur, pH un 1,2-phénylène, qui peut être substitué par au plus deux substituants du groupe constitué par les alcoyles inférieurs, les alcoxy inférieurs, les alcoyle inférieur-thio, les halogènes et le trifluorométhyle, $C_nH_{2n}$ représente un alcoylène inférieur qui sépare les deux atomes d'azote par deux ou trois atomes de carbone, et $R_5$ un hydrogène, un alcoyle inférieur, un carboxy, un carbalcoxy inférieur ou un (hydroxy, amino, mono- ou di-alcoyle inférieuramino)-alcoyle inférieur; les dérivés alcoxy inférieur-carbonyle, alcanoyle inférieur, alcanoyle supérieur, adamantoyle, carbamoyle, mono- ou di-alcoyle inférieur-carbamoyle, cycloalcoyl-carbonyle, ayant de 3 à 7 chaînons, ou les dérivés de HPhCO, les 2-N-oxydes, les dérivés de 2-alcoyle inférieur- ou de 2-HPh-alcoyle inférieur-ammonium quaternaire et leurs sels.

2. Composés de la formule I donnée dans la revendication 1 où chacun des symboles $R_1$, $R_3$ et $R_4$ représente un hydrogène, $R_2$ un hydrogène, un alcoyle inférieur, un alcényle inférieur, un alcynyle inférieur, un hydroxy-alcoyle inférieur, un cycloalcoyle ou un cycloalcoyl-alcoyle inférieur avec à chaque fois de 3 à 7 atomes de carbone dans le noyau, Ph un 1,2-phénylène, un (alcoyle inférieur)-1,2-phénylène, un (alcoxy inférieur)-1,2-phénylène, un (alcoyle inférieur-thio)-1,2-phénylène, un (halogène)-1,2-phénylène ou un (trifluorométhyl)-1,2-phénylène, n le nombre entier 2 ou 3, et $R_5$ un hydrogène, un alcoyle inférieur ou un hydroxy-alcoyle inférieur, leurs dérivés alcanoyle inférieurs, adamantoyle, carbamoyle, mono- ou di-alcoyle inférieur-carbamoyle ou HPhCO, 2-N-oxydes, dérivés 2-alcoyle inférieur ou 2-HPh-alcoyle inférieur quaternaires et leurs sels d'addition acides thérapeutiquement utilisables.

3. Composés de formule générale II

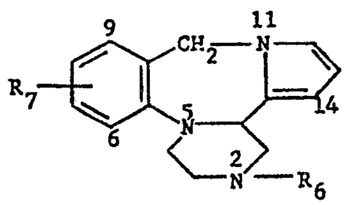

(II)

où $R_6$ représente un hydrogène, un alcoyle inférieur, un alcényle inférieur, un alcynyle inférieur, un hydroxy-alcoyle inférieur ou un cycloalcoyl-alcoyle inférieur ayant de 3 à 7 chaînons, et $R_7$ représente un hydrogène, un halogène ou un trifluorométhyle, et leurs sels d'addition acides thérapeutiquement acceptables.

4. Composés de formule II donnée dans la revendication 3, où $R_6$ représente un alcoyle, un alcényle, un alcynyle ou un hydroxyalcoyle avec à chaque fois jusqu'à 4 atomes de carbone ou un cyclopropylméthyle, et $R_7$ représente un fluor ou un chlore, et leurs sels d'addition acides thérapeutiquement utilisables.

5. Composés de formule II donnée dans la revendication 3, où tous les symboles ont la signification donnée dans la revendication 4, et où $R_7$ est en position 7.

6. 2 - Méthyl - 1,3,4,14b - térahydro - 2H,10H - pyrazino[1,2-a]pyrrolo[2,1-c][1,4]benzodiazépine, ses antipodes d et l, ses 2-N-oxydes et ses sels d'addition acides thérapeutiquement acceptables.

7. 2 - (2 - Hydroxy - éthyl) - 1,3,4,14b - tétrahydro - 2H,10H - pyrazino[1,2-a]pyrrolo[2,1-c][1,4]-benzodiazépine et ses sels d'addition acides thérapeutiquement acceptables.

8. Préparations pharmaceutiques contenant un composé selon l'une des revendications 1 à 7 avec un support pharmaceutique.

9. Composés de formule I de la revendication 1 pour application dans un procédé de traitement thérapeutique du corps de l'homme ou de l'animal.

10. Les composés des revendications 1 à 7 aux fins d'application comme produits pharmaceutiques.

11. Composés de formule I données dans la revendication 1 aux fins d'application comme antidépresseurs, analgésiques et anti-histaminiques.

12. Procédé de préparation de nouvelles 1,2,4,14b-tétrahydro-2H,10H-pyrazino[1,2-a]-pyrrolo[2,1-c][1,4]benzodiazépines selon la formule I de la revendication 1, caractérisé en ce qu'on réduit un composé de formule générale III

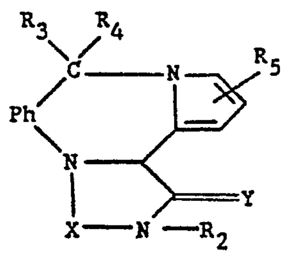

(III)

où X représente un alcoylène inférieur, un mono- ou du dioxo-alcoylène inférieur qui sépare les deux atomes d'azote par 2 ou 3 atomes de carbone et où l'oxo est lié à l'atome de carbone voisin de l'atome d'azote, et Y représente un oxo, deux atomes d'hydrogène ou un hydrogène et un alcoyle inférieur, avec la précision qu'au moins un groupe oxo est présent en X ou Y, et, si on désire un composé où $R_2$ représente un alcoyle inférieur, un alcoyle supérieur, un alcényle inférieur, un alcynyle inférieur, un cycloalcoyle, un cycloalcoyl-alcoyle inférieur, un cycloalcényle, un HPh-alcoyle inférieur ou un carbalcoxy-alcoyle inférieur, dans un composé de formule I où $R_2$ représente un hydrogène, on introduit l'un des radicaux mentionnés, et/ou, lorsqu'on désire un composé où $R_2$ représente un carboxy-alcoyle inférieur, on hydrolyse un composé de formule I où $R_2$ représente un carbalcoxy-alcoyle inférieur, et/ou, lorsqu'on désire un composé où $R_2$ représente un carbamoyl-alcoyle inférieur, un (mono- ou dialcoyle inférieur-carbamoyl)-alcoyle inférieur, dans un produit final où $R_2$ représente un carbalcoxy-alcoyle inférieur, on transforme ce groupe dans un des radicaux mentionnés, et/ou lorsqu'on désire un composé où $R_2$ est un alcanoyle inférieur-alcoyle inférieur ou un HPhCO-alcoyle inférieur, on introduit dans un produit final où $R_2$ représente un hydrogène l'un des radicaux mentionnés, et/ou, lorsqu'on désire un composé où $R_2$ est un (mono- ou dialcoyle inférieur-carbamoyl)-alcoyle inférieur, on alcoyle un produit final où $R_2$ est un carbamoylalcoyle inférieur ou un (mono-alcoyle inférieur-carbamoyl)-alcoyle inférieur, et/ou lorsqu'on désire un composè qui présente en position 2 un radical acyle mentionné ci-dessus, on acyle un produit où $R_2$ représente un hydrogène, et/ou, lorsqu'on désire un produit où $R_2$ représente un alcoxy inférieur-carbonyle, dans un produit final où $R_2$ représente un alcoyle inférieur, un alcényle inférieur ou $—CH_2—HPh$, on transforme ces groupes en un groupe alcoxy inférieur-carbonyle, et/ou, lorsqu'on désire un composé où le substituant sur l'atome d'azote en position 2 et/ou $R_5$ est un alcanoyloxy inférieur-alcoyle inférieur ou un alcanoyloxy supérieur-alcoyle inférieur, on estérifie un produit avec un ou deux groupes hydroxy-alcoyle inférieur, et/ou, lorsqu'on désire un composé où $R_3$ et/ou $R_4$ représente un alcoyle inférieur, on introduit un alcoyle inférieur dans un composé obtenu où $R_3$ et/ou $R_4$ représente un hydrogène, et/ou, lorsqu'on désire un composé où $R_5$ en position 12 est un radical acyl, on cyle un produit final dans cette position, et/ou, lorsqu'on désire un composé où $R_5$ en position 12 est un groupe carboxy, on hydrolyse un produit final où $R_5$ représente un alcoxy inférieur-carbonyle, et, lorsqu'on désire un composé quaternaire, on quaternise un produit substitué en position 2 en introduisant un radical alcoyle inférieur ou HPh-alcoyle inférieur en position 2, et/ou, lorsqu'on désire un N-oxyde, on N-oxyde un produit où l'atome d'azote en position 2 présente un substituant, et/ou, si on le désire, on transforme un composé de formule I obtenu en un autre composé de l'invention, et/ou, si on le désire, on transforme un composé libre obtenu en un sel ou un sel obtenu en le composé libre ou en un autre sel, et/ou, si on le désire, on dédouble un mélange d'isomères ou de racémates obtenu en les isomères ou racémates isolés, et/ou, si on le désire, on dédouble les racémates obtenus en les antipodes optiques.

## Claims

1. A 1,3,4,14b-tetrahydro-2H,10H-pyrazino[1,2-a]pyrrolo[2,1-c][1,4]benzodiazepine of the general formula I

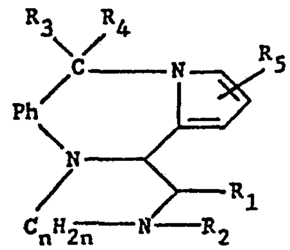

(I)

wherein each of $R_1$, $R_3$ and $R_4$ is hydrogen or lower alkyl; $R_2$ is hydrogen, lower alkyl or higher alkyl, lower alkenyl, lower alkynyl, cycloalkyl or cycloalkenyl, each containing 3 to 7 ring members, or (cycloalkyl, hydroxy, amino, mono- or di-lower alkylamino, carboxy, lower carbalkoxy, carbamoyl, mono- or di-lower alkylcarbamoyl, HPh, lower alkanoyl or HPhCO)-lower alkyl; Ph is 1,2-phenylene which can be substituted by at most two members selected from the group consisting of lower alkyl, lower alkoxy, lower alkylthio, halogen and trifluoromethyl; $C_2H_{2n}$ is lower alkylene separating both nitrogen atoms by 2 or 3 carbon atoms and $R_5$ is hydrogen, lower alkyl, carboxy, lower carbalkoxy or (hydroxy, amino, mono- or di-lower alkylamino)-lower alkyl; the lower alkoxycarbonyl, lower or higher alkanoyl, adamantoyl, carbamoyl, mono- or di-lower alkylcarbamoyl derivates, cycloalkyl-carbonyl derivatives containing 3 to 7 ring members, or HPhCO-derivatives; the 2-N-oxide, 2-lower alkyl or 2-HPh-lower alkyl quaternary ammonium derivatives and salts thereof.

2. A compound of the formula I as indicated in claim 1, wherein each of $R_1$, $R_3$ and $R_4$ is hydrogen; $R_2$ is hydrogen, lower alkyl, lower alkenyl, lower alkynyl, hydroxy-lower alkyl, cycloalkyl or cycloalkyl-lower alkyl, each containing 3 to 7 ring members, Ph is 1,2-phenylene, (lower alkyl)-1,2-phenylene, (lower alkoxy)-1,2-phenylene, (lower alkylthio)-1,2-phenylene, (halogeno)-1,2-phenylene or (trifluoromethyl)-1,2-phenylene; n is 2 or 3, and $R_5$ is hydrogen, lower alkyl or lower hydroxyalkyl; the lower alkanoyl, adamantoyl, carbamoyl, mono- or di-lower alkylcarbamoyl or HPhCO-derivatives; 2-N-oxides; 2-lower alkyl or 2-HPh-lower alkyl quaternary derivatives and the therapeutically useful acid addition salts thereof.

3. A compound of the general formula II,

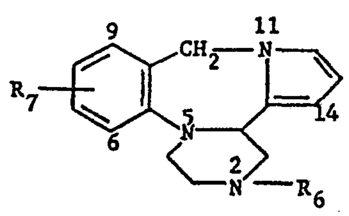

(II)

wherein $R_6$ is hydrogen, lower àlkyl, lower alkenyl, lower alkynyl, hydroxy-lower alkyl or cycloalkyl-lower alkyl containing 3 to 7 ring members and $R_7$ is hydrogen, halogen or trifluoromethyl, and the therapeutically useful acid addition salts thereof.

4. A compound of the general formula II shown in claim 4, wherein $R_6$ is alkyl, alkenyl, alkynyl or hydroxyalkyl, each with up to 4 carbon atoms or cyclopropylmethyl and $R_7$ is fluorine or chlorine, and the therapeutically useful acid addition salts thereof.

5. A compound of the formula II as indicated in claim 3, wherein all the symbols are as defined in claim 4 and $R_7$ is in the 7-position.

6. 2 - Methyl - 1,3,4,14b - tetrahydro - 2H,10H - pyrazino[1,2-a]pyrrolo[2,1-c][1,4]benzodiazepine, and the therapeutically useful acid addition salts thereof.

7. 2 - (2 - Hydroxyethyl) - 1,3,4,14b - tetrahydro - 2H,10H - pyrazino[1,2-a]pyrrolo[2,1-c]-[1,4]benzodiazepine and the therapeutically useful acid addition salts thereof.

8. A pharmaceutical preparation containing a compound according to any one of claims 1 to 7, in conjunction with a pharmaceutical carrier.

9. A compound of the formula I as indicated in claim 1 for use in a therapeutic method of treating humans or animals.

10. A compound of any one of claims 1 to 7 for use as pharmaceutical preparation.

11. A compound of the formula I as indicated in claim 1 for use as antidepressive, analgetic or antihistamine.

12. A process for the manufacture of a 1,3,4,14b-tetrahydro-2H,10H-pyrazino[1,2-a]pyrrolo[2,1-c][1,4]benzodiazepine of the general formula I as indicated in claim 1, characterised in that a compound of the general formula III

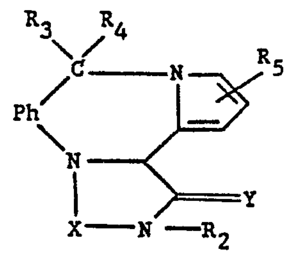

(III),

wherein X is lower alkylene, mono- or dioxo-lower alkylene, which separates both nitrogen atoms by 2 or 3 carbon atoms and wherein oxo is attached to the carbon atoms adjacent to the nitrogen atoms, and Y is oxo, two hydrogens or hydrogen and lower alkyl, with the proviso that at least one oxo group is present in X or Y, is reduced, and, if it is desired to obtain a compound in which $R_2$ is lower alkyl, higher alkyl, lower alkenyl, lower alkynyl, cycloalkyl, cycloalkyl-lower alkyl, HPh-lower alkyl or carbalkoxy-lower alkyl, such a radical is introduced into a compound of the formula I, in which $R_2$ is hydrogen, and/or, if it is desired to obtain a compound, in which $R_2$ is carboxy-lower alkyl, a compound of the formula I, wherein $R_2$ is carbalkoxy-lower alkyl, is hydrolysed, and/or, if it is desired to obtain a compound in which $R_2$ is carbamoyl-lower alkyl, (mono- or di-lower alkylcarbamoyl)-lower alkyl, such a group is converted into one of the radicals specified above in a final product in which $R_2$ is carbalkoxy-lower alkyl, and/or, if it is desired to obtain a compound in which $R_2$ is lower alkanoyl-lower alkyl or HPhCO-lower alkyl, one of the above radicals is introduced into a final product in which $R_2$ is hydrogen, and/or, if it is desired to obtain a compound in which $R_2$ is (mono- or di-lower alkylcarbamoyl)-lower alkyl, a final product in which $R_2$ is carbamoyl-lower alkyl or (mono-lower alkylcarbamoyl)-lower alkyl, is alkylated, and/or, if it is desired to obtain a compound containing an acyl radical referred to above in the 2-position, a product in which $R_2$ is hydrogen is acylated, and/or, if it is desired to obtain a compound in which $R_2$ is lower alkoxycarbonyl, in a final product in which $R_2$ is lower alkyl, lower alkenyl or $-CH_2-HPh$, these groups are converted into a lower alkoxycarbonyl group, and/or, if it is desired to obtain a compound in which the substituent at the nitrogen atom in the 2-position and/or $R_5$ is a lower alkanoyloxy-lower alkyl or a higher alkanoyloxy-lower alkyl radical, a product containing one or two hydroxy-lower alkyl groups is esterified, and/or, if it is desired to obtain a compound in which $R_3$ and/or $R_4$ is lower alkyl, lower alkyl is introduced into a resultant compound in which $R_3$ and/or $R_4$ is hydrogen, and/or, if it is desired to obtain a compound in which $R_5$ in the 12-position is an acyl radical, a final product is acylated in this position, and/or, if it is desired to obtain a compound in which $R_5$ in the 12-position is a carboxyl group, a final product in which $R_5$ is lower alkoxycarbonyl is hydrolysed, and, if it is desired to obtain a quaternary compound, a product which is substituted in the 2-position is quaternised by introducing a lower alkyl or HPh-lower alkyl radical in the 2-position, and/or, if it is desired to obtain a N-oxide, a product in which the nitrogen atom in the 2-position contains a substituent is N-oxidised, and/or, if desired, a resultant compound of the formula I is converted into another compound of the invention, and/or, if desired, a resultant salt is converted into the free compound or into another salt, and/or, if desired, a resultant mixture of isomers or racemates is resolved into the individual isomers or racemates, and/or, if desired, resultant racemates are resolved into the optical antipodes.